(19) Europäisches Patentamt
European Patent Office
Office européen des brevets

(11) **EP 3 382 584 A1**

(12) **EUROPEAN PATENT APPLICATION**

(43) Date of publication:
**03.10.2018 Bulletin 2018/40**

(51) Int Cl.:
***G06F 19/00*** *(2018.01)*

(21) Application number: **17164007.1**

(22) Date of filing: **30.03.2017**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(71) Applicant: **FUJITSU LIMITED
Kawasaki-shi,
Kanagawa 211-8588 (JP)**

(72) Inventors:
• **VILLAZON-TERRAZAS, Boris
28003 Madrid (ES)**

• **GARCIA SANTA, Nuria
28010 Madrid (ES)**
• **DE LA TORRE, Victor
28007 Madrid (ES)**

(74) Representative: **Haseltine Lake LLP
Lincoln House, 5th Floor
300 High Holborn
London WC1V 7JH (GB)**

Remarks:
Amended claims in accordance with Rule 137(2)
EPC.

(54) **A SYSTEM AND A METHOD TO PREDICT PATIENT BEHAVIOUR**

(57)    A system arranged to predict patient clinical behaviour, the processor being arranged to provide: a patient clinical object, PCO, engine to accept historical clinical behaviour of each patient in a population of patients and to create a PCO for each patient linking a patient identification with a clinical node for each historical piece of clinical information for that patient; a patient activity object, PAO, engine to input historical activity behaviour of each patient in the population of patients in the form of pieces of activity information identifying one or more visits to healthcare facilities; and to create a PAO for each patient linking the patient identification with an activity node for each historical piece of activity information for that patient; a clinical pattern miner, to use machine learning on the patient population PCOs to cluster the patients into two or more clinical patterns according to the PCO of each of the population of patients; a patient behaviour estimator, to: input the PAO and PCO for each of the population of patients clustered within each clinical pattern and to use machine learning to create a model of the clinical patterns; to input activity behaviour of a new patient in the form of pieces of activity information as to any visits of the new patient to healthcare facilities; to create a PAO for the new patient linking the new patient identification with an activity node for each piece of activity information for the new patient; to use machine learning to classify the new patient into one of the clinical patterns ; and to predict new patient clinical behaviour from the clinical nodes of the clinical pattern in which the new patient has been classified.

FIG. 1

**Description**

This invention relates to prediction of clinical behaviour of an individual or a living subject, usually referred to as a patient, with a medical condition. The patient may be a human or potentially an animal, such as a specimen of a rare breed or even a pet. In many scenarios, the patient may also be suffering from a disorder, but in others the patient is currently healthy, and the term medical condition includes conditions such as pregnancy, as well as disorders, illnesses and diseases. The invention is thus applicable in medicine, healthcare and veterinary science.

Within a healthcare system the behaviour of patients may be represented from two points of view/perspectives, namely, (1) the clinical view, which includes the symptoms, treatments, diagnoses and drugs of patients, and (2) the activity view, which includes different visits to the hospital units, for example, the patient A visited the Emergency Room on November 2016, 18th at 16:00.

Modelling and estimating future patient behaviour (also known as a patient pathway) in health care systems is considered vital in understanding health system activity and thus in improving its functionality. One challenge is to define a good set of features that are able to represent the temporal aspect of electronic health records (EHR). The clinical variables of the EHR are acquired asynchronously, which means they are measured at different time moments.

Modelling and predicting the behaviour of the patients is not a straightforward task. There is a lack of models and mechanisms to model and predict a patient's next steps within a healthcare system. This prediction is very important for functionality of healthcare systems. Prediction is important also for insurance companies, because those companies are willing to incentive and promote the clients with fewer medical incidents.

Health insurance is insurance against the risk of incurring medical expenses among individuals. By estimating the overall risk of health care and health system expenses, among a targeted group, an insurer can develop a routine finance structure, such as a monthly premium or payroll tax, to ensure that money is available to pay for the health care benefits specified in the insurance agreement. Hence, health insurance companies would also like to know the potential medical/clinical behaviour of their clients, so the companies can increase/decrease the client policies according to this behaviour.

According to an embodiment of a first aspect of the invention, there is provided a system comprising a processor and memory arranged to predict patient clinical behaviour, the processor being arranged to provide:

> a patient clinical object, PCO, engine to accept historical clinical behaviour of each patient in a population of patients in the form of pieces of clinical information identifying one or more symptoms, treatment, diagnoses and drugs including dates of the symptom, treatment, diagnosis or drug, and to create a PCO stored in the memory for each patient linking a patient identification with a clinical node for each historical piece of clinical information for that patient;
> a patient activity object, PAO, engine to input historical activity behaviour of each patient in the population of patients in the form of pieces of activity information identifying one or more visits to healthcare facilities, including the type of the healthcare facility visited and the date of the visits; and to create a PAO stored in the memory for each patient linking the patient identification with an activity node for each historical piece of activity information for that patient;
> a clinical pattern miner, to use machine learning on the patient population PCOs stored in the memory to cluster the patients into two or more clinical patterns according to the PCO of each of the population of patients;
> a patient behaviour estimator, to:

>> input from the memory the PAO and PCO for each of the population of patients clustered within each clinical pattern and to use machine learning to create a model of the clinical patterns, the model including clinical nodes and activity nodes from patients clustered within each clinical pattern; to
>> input activity behaviour of a new patient in the form of pieces of activity information as to any visits of the new patient to healthcare facilities, including the type of the healthcare facility visited and the date of the visits; to create a PAO for the new patient linking the new patient identification with an activity node for each piece of activity information for the new patient; to
>> use machine learning to classify the new patient into one of the clinical patterns by comparing the activity nodes in the new patient PAO with the activity nodes in the pattern; and to
>> predict new patient clinical behaviour from the clinical nodes of the clinical pattern in which the new patient has been classified.

The PCO and PAO for each patient in the population of patients are linked and together essentially provide all the clinical and activity (visit) behaviour of that patient, for example from an EHR and as categorised in the EHR. The clinical pattern miner clusters the patients into clusters (groups) according to their clinical behaviour. The patient behaviour estimator inputs the PAO and PCO (all the clinical nodes and all the activity nodes) for each of the population of patients clustered within each clinical pattern. The nodes for each patient are of course linked together by patient identification and potentially patient clinical information. The patient behaviour estimator then uses machine learning to create a model of the clinical patterns, the model including clinical nodes and activity nodes from patients clustered within each clinical pattern. A PAO with activity behaviour of a new patient can be compared with the activity nodes in each pattern, to classify the new patient PAO into a pattern and to predict new patient clinical behaviour from the clinical nodes of the clinical pattern in which the new patient has been classified. The predicted clinical behaviour is taken from the predicted nodes in the clinical pattern. The name of the pattern may also be provided to the system user as an overall indication of the pattern.

Hence embodiments of the invention aim to predict a patient behaviour from the clinical (and potentially also the activity) point of view, and can rely exclusively on activity behaviour of a new patient, without any input of clinical information. For the avoidance of doubt, the term input includes both input from a separate system or from memory and manual input.

In other words, invention embodiments are able to predict the clinical (and activity) behaviour of patients. To this end, the invention relies on clinical and activity data of a population of patients, i.e., all the symptoms, treatments, diagnoses and drugs, along with hospital visits and visits to specific units inside or outside the hospital or other healthcare facility. With the resulting predictions, the healthcare system is able to manage the resources in a much better way. Moreover, patients will get better treatment on time. The same predictions can be used in an academic medical centre, for research on healthcare systems or even improved diagnosis/treatment.

The clinical pattern miner may be arranged to use a biomedical knowledge graph, the biomedical knowledge graph including symptoms, drugs, treatments, diagnoses and diseases, and links between the symptoms, drugs, treatments, diagnoses, and diseases taken from theoretical and empirical knowledge. The clinical pattern miner may use the biomedical knowledge graph to supplement information from the population of patients.

For example, the clinical pattern miner may be arranged to supplement nodes on the patient PCOs to provide supplemented PCOs including additional clinical nodes taken from the biomedical knowledge graph, by adding neighbouring nodes from the biomedical knowledge graph to PCO nodes which are already present on both the biomedical knowledge graph and the PCO. Since the biomedical knowledge graph is viewed as encoding many more relations than a single patient, each supplemented PCO may be viewed as a subgraph of the biomedical knowledge graph.

The biomedical knowledge graph may include link weights (for example from 0 to 1) on the links between its entities and in this case, a neighbouring node with a link to a node that is already present with a weight above a threshold (say 0.5) may become an additional clinical node.

The patient behaviour estimator may be arranged to input the additional clinical nodes for each of the population of patients clustered within each clinical pattern (as well as the activity nodes and clinical nodes), wherein each clinical pattern includes the additional clinical nodes from patients clustered within the clinical pattern. Hence the data from the biomedical knowledge graph is used in predicting the behaviour of a new patient.

The patient behaviour estimator may be arranged to label each of the clinical nodes and activity nodes from patients clustered within a clinical pattern with a node weight which is derived from the proportion of patients within the population that include the node and that are clustered within the pattern.

The patient behaviour estimator may include a meta predictor arranged to use the nodes (and potentially also the weights for nodes) of the pattern included within the new patient PAO to derive a combined probability of classification within the pattern.

For example, the combined probability may be derived by categorising the nodes into diagnoses, drugs, symptoms, treatments and visits and by weighting the contribution of each category with an assigned weight for that category.

The system may further comprise an activity pattern miner, to use machine learning on the patient population PAOs to cluster the patients into two or more activity patterns according to the PAO of each of the population of patients; wherein the patient behaviour estimator is additionally arranged to:

use machine learning to create a model of the activity patterns, the model including clinical nodes and activity nodes from patients clustered within each activity pattern; to
use machine learning to classify the new patient into one of the activity patterns by comparing the activity nodes in the new patient PAO with the activity nodes in the pattern; and to
predict new patient activity behaviour from the activity nodes of the pattern in which the new patient has been classified.

Thus the same system and same historical data and new patient data may be used to predict new patient behaviour.

There may also be clinical behaviour data available for the new patient which could be used in classification into a clinical or activity pattern. The patient behaviour estimator may thus be additionally arranged to:

input clinical behaviour of a new patient in the form of pieces of clinical information identifying one or more symptoms, treatment, diagnoses and drugs including dates of the symptom, treatment, diagnosis or drug; and to create a PCO for the new patient linking the new patient identification with a clinical node for each piece of clinical information for the new patient; and to
additionally use the clinical nodes of the new patient PCO to classify the new patient into a (clinical or activity) pattern, by additionally comparing the (clinical activity) nodes in the new patient PCO with the clinical nodes in the pattern.

In this case, the meta predictor may be further arranged to additionally use the nodes/node weights for nodes of the pattern included within the new patient PCO to derive the combined probability of classification within the pattern.

According to an embodiment of a second aspect of the invention, there is provided a computer-implemented method of predicting patient clinical behaviour comprising:

inputting historical clinical behaviour of each patient in a population of patients in the form of pieces of clinical information identifying one or more symptoms, treatment, diagnoses and drugs including dates of the symptom, treatment, diagnosis or drug, and creating a PCO for each patient linking a patient identification with a clinical node for each historical piece of clinical information for that patient;
inputting historical activity behaviour of each patient in the population of patients in the form of pieces of activity information identifying one or more visits to healthcare facilities, including the type of the healthcare facility visited and the date of the visits; and creating a PAO for each patient linking the patient identification with an activity node for each historical piece of activity information for that patient;
using machine learning on the patient population PCOs to cluster the patients into two or more clinical patterns according to the PCO of each of the population of patients;
inputting the PAO and PCO for each of the population of patients clustered within each clinical pattern and using machine learning to create a model of each clinical pattern, the model including clinical nodes and activity nodes from patients clustered within the clinical pattern;
inputting activity behaviour of a new patient in the form of pieces of activity information as to any visits of the new patient to healthcare facilities, including the type of the healthcare facility visited and the date of the visits;
creating a PAO for the new patient linking the new patient identification with an activity node for each piece of activity information for the new patient;
using machine learning to classify the new patient into one of the clinical patterns by comparing the activity nodes in the new patient PAO with the activity nodes in the pattern; and
predicting new patient clinical behaviour from the clinical nodes of the clinical pattern in which the new patient has been classified.

A method according to preferred embodiments of the present invention can comprise any combination of the apparatus aspects. Methods or computer programs according to further embodiments can be described as computer-implemented in that they require processing and memory capability.

The apparatus according to preferred embodiments is described as configured or arranged to, or simply "to" carry out certain functions. This configuration or arrangement could be by use of hardware or middleware or any other suitable system. In preferred embodiments, the configuration or arrangement is by software.

Thus according to one aspect there is provided a program which, when loaded onto at least one computer configures the computer to become the system according to any of the preceding apparatus definitions or any combination thereof.

According to a further aspect there is provided a program which when loaded onto the at least one computer configures the at least one computer to carry out the method steps according to any of the preceding method definitions or any combination thereof.

In general the computer may comprise the elements listed as being configured or arranged to provide the functions defined. For example this computer may include memory, processing, and a network interface.

The invention can be implemented in digital electronic circuitry, or in computer hardware, firmware, software, or in combinations of them. The invention can be implemented as a computer program or computer program product, i.e., a computer program tangibly embodied in a non-transitory information carrier, e.g., in a machine-readable storage device, or in a propagated signal, for execution by, or to control the operation of, one or more hardware modules.

A computer program can be in the form of a stand-alone program, a computer program portion or more than one computer program and can be written in any form of programming language, including compiled or interpreted languages, and it can be deployed in any form, including as a stand-alone program or as a module, component, subroutine, or other unit suitable for use in a data processing environment. A computer program can be deployed to be executed on one module or on multiple modules at one site or distributed across multiple sites and interconnected by a communication network.

Method steps of the invention can be performed by one or more programmable processors executing a computer program to perform functions of the invention by operating on input data and generating output. Apparatus of the invention can be implemented as programmed hardware or as special purpose logic circuitry, including e.g., an FPGA (field programmable gate array) or an ASIC (application-specific integrated circuit).

Processors suitable for the execution of a computer program include, by way of example, both general and special purpose microprocessors, and any one or more processors of any kind of digital computer. Generally, a processor will receive instructions and data from a read-only memory or a random access memory or both. The essential elements of a computer are a processor for executing instructions coupled to one or more memory devices for storing instructions and data.

The invention is described in terms of particular embodiments. Other embodiments are within the scope of the following claims. For example, the steps of the invention can be performed in a different order and still achieve desirable results. Multiple test script versions can be edited and invoked as a unit without using object-oriented programming technology; for example, the elements of a script object can be organized in a structured database or a file system, and the operations described as being performed by the script object can be performed by a test control program.

Elements of the invention have been described using the terms "patient clinical object, PCO, engine", "patient activity object, PAO, engine", "pattern miner" and "patient behaviour estimator" etc. The skilled person will appreciate that such functional terms and their equivalents may refer to parts of the system that are spatially separate but combine to serve the function defined. Equally, the same physical parts of the system may provide two or more of the functions defined.

For example, separately defined means may be implemented using the same memory and/or processor as appropriate.

Preferred features of the present invention will now be described, purely by way of example, with references to the accompanying drawings, in which:-

Figure 1 is a block diagram of main system components in a general embodiment of the invention;
Figure 2 is a flow chart of a method in a general embodiment;
Figure 3 is a block diagram of main system components in a further embodiment;
Figure 4 is a block diagram of a biomedical knowledge graph engine;
Figure 5 is an example of a biomedical theoretical knowledge graph;
Figure 6 is an example of a biomedical empirical knowledge graph;
Figure 7 is an example of a biomedical knowledge graph;
Figure 8 is a block diagram of a patient behavioural pattern engine;
Figure 9 is an example of a PCO in graph and timeline form;
Figure 10 is an example of a PAO in graph and timeline form;
Figure 11 is a block diagram of inputs and outputs of a clinical pattern miner;
Figure 12 is an example of a clinical pattern miner with a resultant grouping of patients;
Figure 13 is a block diagram of inputs and outputs of an activity pattern miner;
Figure 14 is an example of an activity pattern miner with a resultant grouping of patients;
Figure 15 is a block diagram of a patient behaviour estimator;
Figure 16 shows an example of a clinical behaviour estimator with patient data and its output;
Figure 17 shows the classification of a patient into one of two clinical groups;
Figure 18 shows an example of an activity behaviour estimator with patient data and its output;
Figure 19 shows the classification of a patient into one of two activity groups;
Figure 20 shows the classification of a patient into one of two clinical groups, using only patient activity data;
Figure 21 is a block diagram showing a hardware configuration for open data retrieval; and
Figure 22 is a block diagram of suitable hardware for implementation of invention embodiments.

Invention embodiments split patient behaviour into two main parts:

- Clinical behaviour, which includes the symptoms, treatments, diagnoses, and drugs of a patient (it should be noted that treatment does not include drugs in this context, but non-drug treatment, such as physiotherapy, occupational therapy, counselling, massage etc.)
- Activity behaviour, which includes the visits of the patient to the different hospital areas (or other healthcare areas)

It is important to mention for the insurance company example that insurance companies only have access to the activity behaviour of a patient. Embodiments of the invention are able to predict the clinical behaviour by only having the activity behaviour. This is aided in some embodiments by use of a theoretical biomedical knowledge graph, and more importantly on its combination with an empirical biomedical knowledge graph based on real patient data. These graphs are described in more detail later.

Also in terms of improving management of resources, it is advantageous that with a limited dataset (for example just the activity behaviour or just the clinical behaviour) it is possible to predict the patient's next step within a healthcare system. Hence the invention embodiments function also under sub-optimal data conditions.

In summary, invention embodiments may

- identify behavioural (clinical and activity) patterns from the patient electronic health record data of a population of patients,
- build a model that is able to predict/estimate when a new patient may be classified in one (or more) of the identified behavioural (clinical and activity) patterns, based on his/her Electronic Health Record (EHR),
- understand which the main drivers behind those behaviours are (the term "drivers" refers to the potential symptoms, treatments, diagnoses, and drugs and hospital visits that are defining the behaviour).

Invention embodiments implement precision medicine based on individual data for a new patient as well as on a bulk of historical data from other patients. This is an emerging approach for disease diagnosis, treatment and prevention that takes into account individual variability in genes, physiology, anatomy, environment, and lifestyle. Precision medicine represents a radically different approach to improve the current clinical workflows and to predict patient behaviour (also known as patient pathway) within the healthcare ecosystem.

## Background

As previously mentioned, estimating and predicting how a particular patient will behave within the healthcare system is very important for hospitals, medical centres and insurance companies in general.

Existing efforts associated with patient pathways do not consider the distinction between clinical and activity behaviour, do not focus on the patient's individual characteristics or do not predict/estimate the upcoming pathways/behaviours of the patient.

## General Embodiment

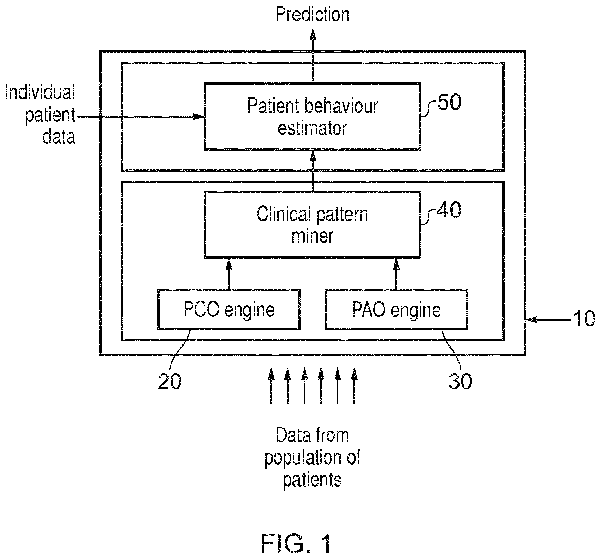

Figure 1 is a block diagram of a system 10 according to a general invention embodiment.

The system includes a PCO (Patient Clinical Object) Engine 20, which creates a PCO for each of a population of patients - for example using EHRs from one or more hospitals. The EHR may classify clinical data into categories of symptoms, treatments, diagnoses and drugs. This "Patient Clinical Object" is a patient health profile/patient health data set, preferably in the form of a semantically rich (structured) aggregation of clinical entities that encapsulates information about a given patient. The PCO contains information about the patient, such as age and sex, and its clinical data, such as (1) symptoms, (2) treatment, (3) diagnoses, and (4) drugs.

The PCO may be represented as a graph, whose nodes include the corresponding timestamps for each one of the clinical features of a patient. All the nodes are linked to the patient identity, which may be viewed as a central node. The clinical features are nodes of a relational data structure, which may be in graph form or in a matrix format or any other suitable format.

The PAO Engine 30 creates a PAO (Patient Activity Object) for each of the population, which is a patient activity profile/patient activity data set and includes the hospital (or other healthcare) visits of the patient to the different hospital/healthcare areas along with the corresponding timestamps. Again, the PAO may be represented as a graph, whose nodes include the corresponding timestamps for each one of the activity features of a patient. All the nodes are linked to the patient identity, which may be viewed as a central node. The activity features are nodes of a relational data structure, which may be in graph form or in a matrix format or any other suitable format.

The system can provide a temporal view representation of such PCOs and PAOs. Thanks to the temporal information, the system can identify/extract temporal patterns (e.g, patient clinical patterns, and/or patient activity patterns) for the population of patients. The clinical pattern miner 40 extracts clinical behaviour patterns using machine learning, for example unsupervised machine learning. Each PCO can be classified into one of these patterns.

Finally, in patient behaviour estimator 50, the system can use machine learning to create a model including each clinical pattern. The model is made up of the patterns with their clinical nodes and activity nodes. This allows the estimator to predict/estimate patient clinical behaviour (in the form of symptoms, treatments, diagnoses and drugs) even when only the activity behaviour of an individual new patient (not one of the previous population) is available.

The individual patient may be classified in one (or more) identified/extracted patient behavioural (clinical and activity) patterns, and the user may be provided with an indication (for example on a GUI) of the main drivers behind those behaviours.

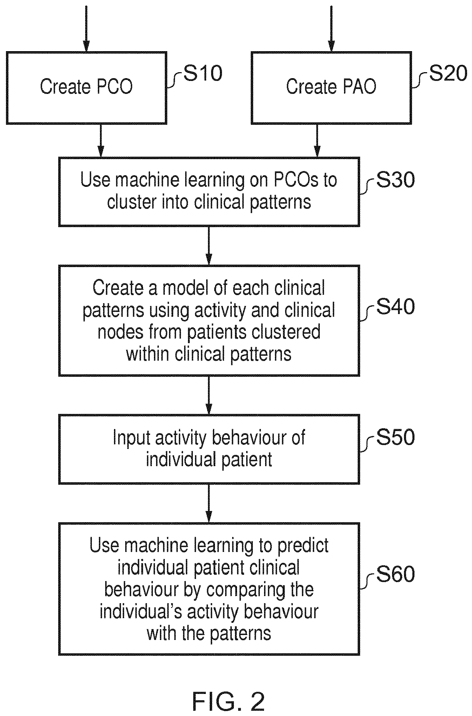

Figure 2 is a flowchart of a method according to a general invention embodiment. In step S10 the PCO is created and probably in parallel, step S20 creates the PAO for each of a population of patients.

In step S30 machine learning is used on the PCO data to cluster the patients into clinical patterns. In step S40 a model is created of the clinical patterns using activity and clinical nodes from patients clustered within each clinical pattern. In step S50 there is an input (manual using a GUI or by the system) of activity behaviour of an individual new patient. Of course, clinical behaviour can also be input at this stage if appropriate.

Step S60 uses machine learning to predict individual patient clinical behaviour by comparing the individual's activity behaviour (and clinical behaviour if available) with the patterns within the model.

**General Description**

The following detailed embodiment relates to a system which can be used in a number of different ways: not only to predict clinical behaviour from activity behaviour, but to predict future activity and/or clinical behaviour from historical activity and/or clinical behaviour.

The system consists of three main modules:

- A first module includes a pattern miner for extracting/identifying the available behavioural (clinical and activity) patterns from the patient electronic health records. Many PCOs and PAOs (each for a single patient in a population of patients) are used to create many temporal representations, one for each patient. Then the patients are divided into groups according to the content of their clinical behaviour (PCO) and/ or activity behaviour (PAO).

- A second module is for creating a biomedical knowledge graph, based on theoretical and empirical knowledge. The biomedical knowledge graph is a knowledge base for representing health related concepts. It encodes theoretical knowledge, but is enhanced with empirical knowledge. Use of the biomedical knowledge graph increases accuracy of results of the system.

- A third module is for predicting/estimating patient behaviour of an individual patient, based on data from the individual patient as well as behavioural (clinical and/or activity) patterns and optionally the biomedical concept relations extracted from the Biomedical Knowledge Graph

Typically, the user selects an individual patient by one or more identifiable attributes (e.g. names, IDs, etc.), and triggers a patient behaviour function supported by client software. This function may generate a list of potential patient behaviour groups each associated with a numeric value, e.g. a weight between 0 and 1 or 0% and 100% indicating the likelihood that the individual patient is associated with that group.

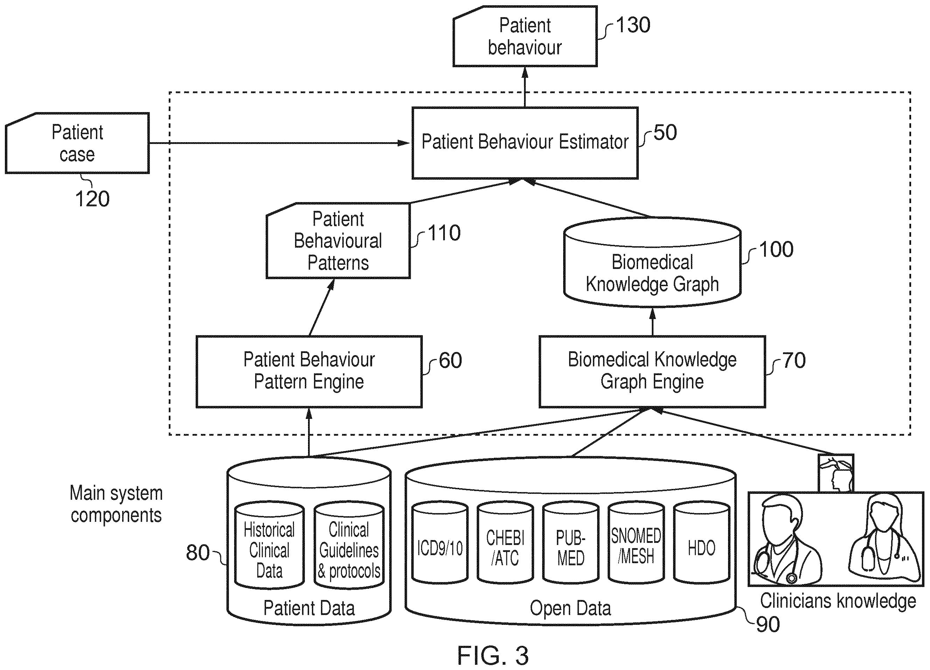

The software running on above system includes three main modules depicted in Figure 3, and they are:

the patient behavioural pattern engine, 60, which has a final goal to extract/identify a set of behavioural (clinical and activity) patient patterns 110;

the biomedical knowledge graph engine, 70, which builds a biomedical knowledge graph 100 that encodes (i) theoretical knowledge extracted from scientific literature and health standards, and (ii) empirical knowledge extracted from real patient electronic health records; and

the patient behaviour estimator, 50, which predicts/estimates the potential behaviours 130 for a new patient case 120, taking as input the identified/extracted patient behavioural patterns 110 and the relations from the biomedical knowledge graph 100.

Patient data 80 may be used in both the patient behavioural pattern engine 60 and the biomedical knowledge graph engine 70. Open data 90 is collected from open sources as described in more detail below for use in the biomedical knowledge graph engine 70. Clinician's knowledge may be another input into the biomedical knowledge graph engine 70.

**Biomedical Knowledge Graph Engine 70**

This engine is for building a knowledge graph that encodes:

- Knowledge from the scientific medical literature, e.g., PubMed (PubMed is a service of the US National Library of Medicine® that: provides free access to MEDLINE®, the NLM® database of indexed citations and abstracts to medical, nursing, dental, veterinary, health care, and preclinical sciences journal articles), and available health standards, such as ICD9 and ICD10, (the ninth and tenth revisions of the International Classification of Diseases, see http://www.icd9data.com/ and http://www.icd10data.com/), CHEBI ("Chemical Entities of Biological Interest" which is a database and ontology of molecular entities, see https://www.ebi.ac.uk/chebi/), ATC (Anatomical Therapeutic Chemical Classification), SNOMED CT (a standardised, multilingual vocabulary of terms relating to the care of the individual), MESH (the U.S. National Library of Medicine's controlled vocabulary thesaurus used for indexing articles for MEDLINE, see https://www.nlm.nih.gov/mesh/), and HDO (a disease ontology, see http://disease-ontology.org/) among others.

- Knowledge from empirical evidence, e.g., connections between medical entities (symptoms, treatments, diagnoses and drugs) based on patient electronic health records (not necessarily the same population as is used to build the PCOs and PAOs), and potentially also on clinical guidelines and protocols.

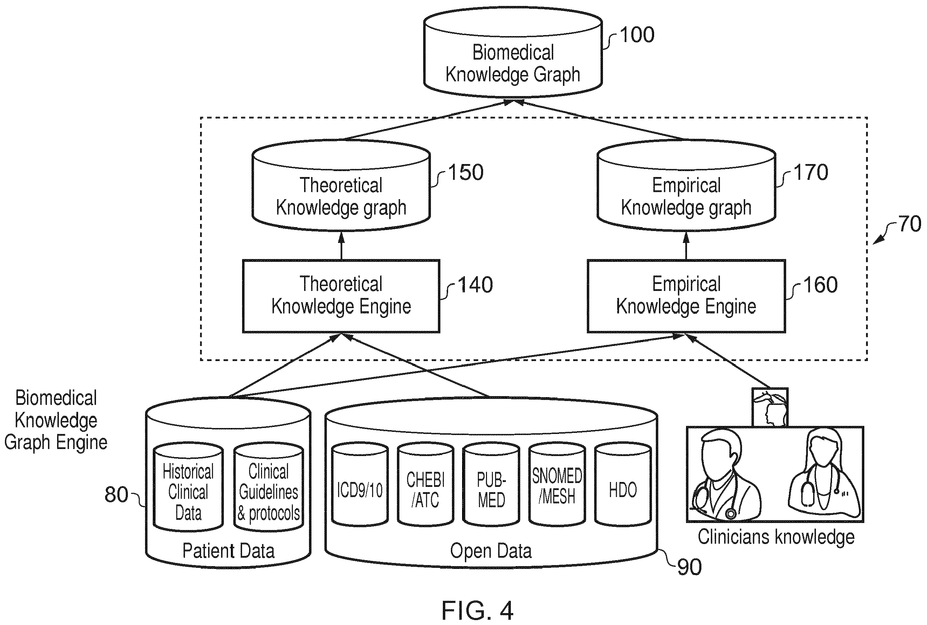

Figure 4 depicts the main subcomponents of this module. The module is arranged with a separate theoretical knowledge engine 140 and empirical knowledge engine 160, providing a theoretical knowledge graph 150 and empirical knowledge graph 170 respectively.

The knowledge graph engines may function in any appropriate way. They may be configured to collect seeds (for example in the form of suggested terms) for an initial set of medical terms and relations between the seed terms. Once the two separate knowledge graphs are created they may be reconciled. This allows a both generic and practically focussed set of terms which is widely applicable and thus of better quality for the following process.

The biomedical knowledge graph engine creates relations between the two knowledge graphs.

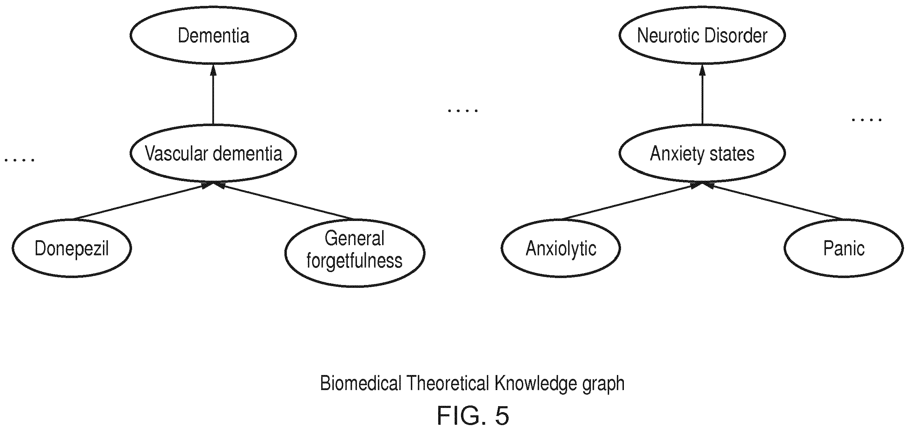

Figure 5 presents an excerpt of the theoretical knowledge graph that shows relations among drugs (lower left node of the two subgraphs depicted), diagnosis (top two nodes), and symptoms (lower right node). Treatment nodes are not shown in this excerpt. All the information is extracted from scientific medical publications, healthcare standards, and clinical guidelines and protocols. For example, different diagnoses may be linked in the graph, because they are both included in a published article, or two diagnoses may be related because the same drug is prescribed for both. Links between the nodes may be weighted according to co-occurrence of the linked terms across the whole data set.

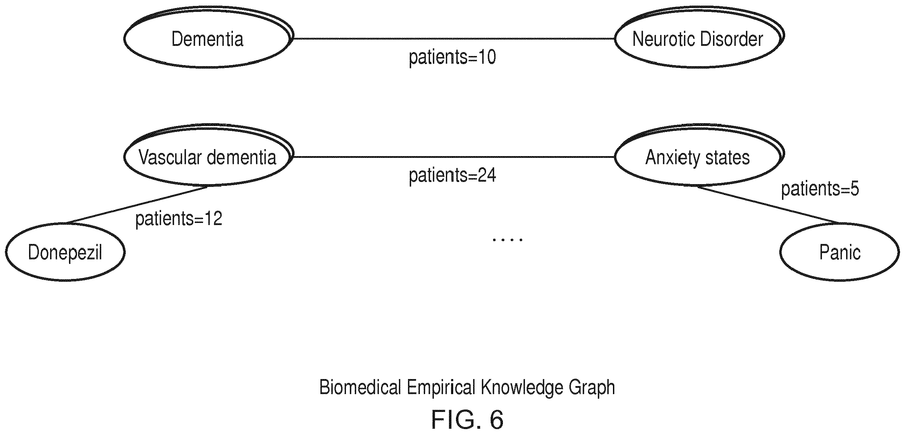

Figure 6 presents an excerpt of the empirical knowledge graph that shows relations among drugs (lower left node), diagnosis (top four nodes), and symptoms (lower right node). The relations represent the number of patients that have such evidence. For example between "dementia" and "neurotic disorder" there are 10 patients that had both diagnoses; as well as "vascular dementia" and "Donepezil", there are 12 patients with "vascular dementia" that have been medicated with "Donepezil".

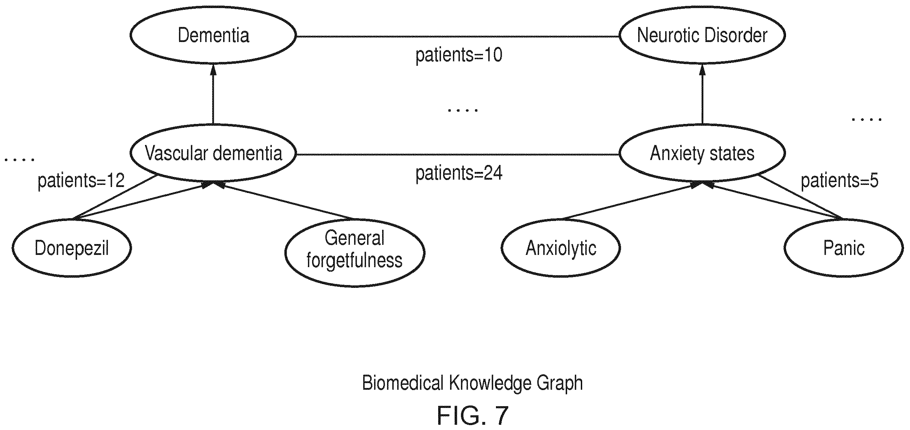

Following the excerpts, the two graphs are combined and give the graph depicted in Figure 7. The links in the resultant graph are shown with details of the number of patients only, but a final weight can incorporate weighting from both constituent graphs.

## Patient Behavioural Pattern Engine 60

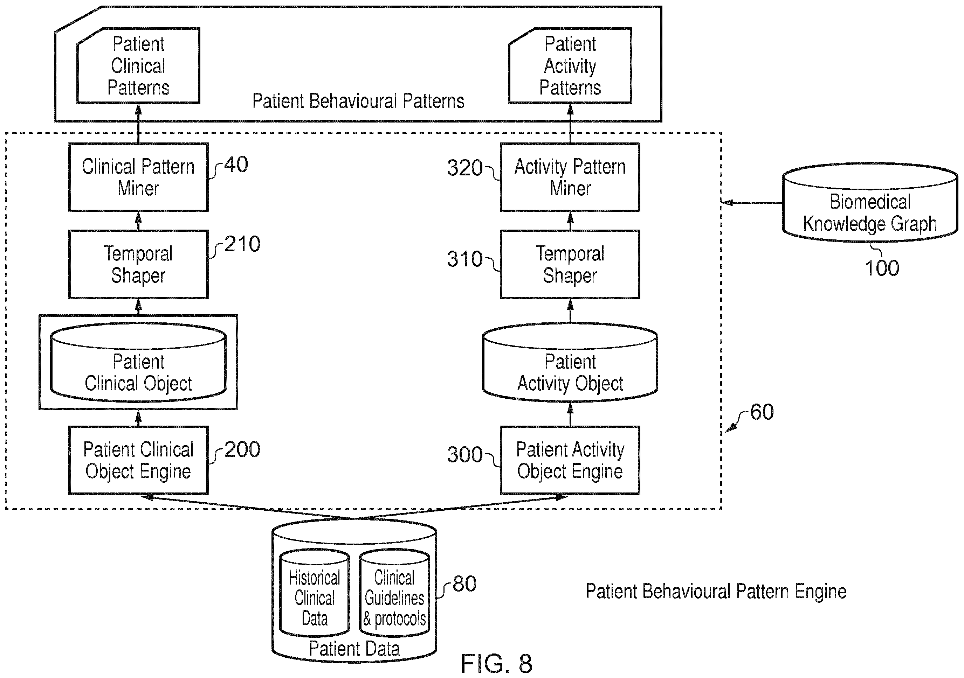

This module identifies/extracts the patient behavioural (clinical and activity) patterns from the Historical Clinical Data. The components are depicted in Figure 8, and they are as follows.

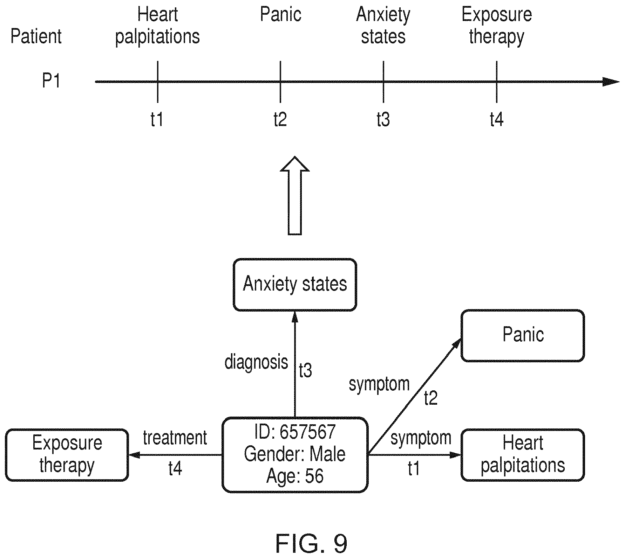

The **Patient Clinical Object Engine** 200 builds the PCO (Patient Clinical Object). The PCO includes the corresponding timestamps (dates) for the health features. It is worth noting that the PCO can be represented as a graph, or as a time line, or in any other suitable form, such a relational data table. Figure 9 depicts a PCO, in two different representations. The graph form is centred on patient information (including an ID, gender and age). Each clinical node links to this central node and is categorised as a symptom, treatment, diagnosis or drug node (perhaps in accordance with the category in an EHR), with a linked time t1-t4. This simple example includes 4 clinical nodes. The alternative representation shown at the top of Figure 9 is a temporal representation which structures the information in time order, using temporal shaper 210 of Figure 8. The symptom of "heart palpitations" for patient P1 at t1 appears first, followed by the clinical behaviour at t2, t3 and then t4.

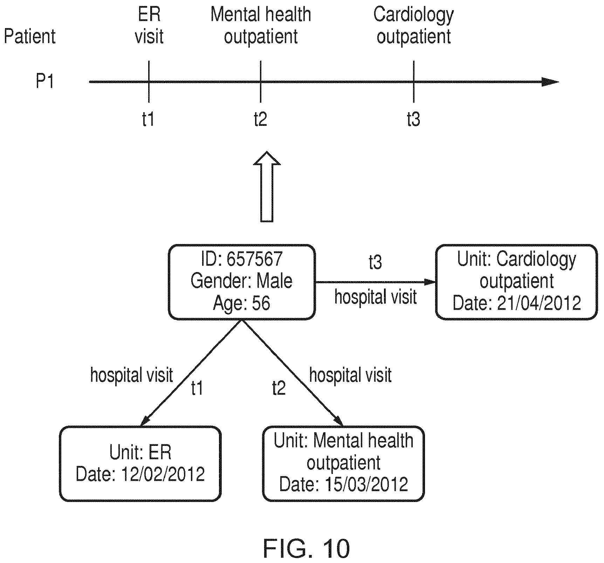

**The Patient Activity Object Engine** 300 builds the PAO (Patient Activity Object). The PAO will include the hospital visits of the patient to the different hospital areas along with the corresponding timestamps (dates). Like the PCO, the PAO can be represented as a graph, or as a time line, or in any other suitable form, such a relational data table. Figure 10 depicts a PAO of the same patient represented in the PCO, in two different representations. The graph form is centred on the patient information (including an ID, gender and age). Each activity node links to this central node and is categorised as a hospital visit, or a hospital visit of a particular type, such as ER (Emergency Room) MH (Mental Health) or cardiology visit (perhaps in accordance with the category in an EHR), with a linked time t1-t4. This simple example includes 3 clinical nodes. The alternative representation shown at the top of Figure 10 is a temporal representation which structures the information in time order, using temporal shaper 310 of Figure 8. The hospital visit to ER for patient P1 is at t1, followed by the mental health visit at t2, and the cardiology visit at t3. Of course the PCO and PAO can be combined, and they are linked by the patient identity.

**The Temporal Shaper** 210/310 transforms the graph representation of the PCO and PAO into a temporal representation, by structuring the information in time order. This representation facilitates the upcoming tasks of pattern extraction.

**The Clinical Pattern Miner** 40 extracts/identifies the patient clinical patterns from a set of patients. Basically, this component, by relying on machine learning techniques, extracts and identifies a set of patient clinical patterns, i.e., classifies patients based on patient clinical features by clustering them into clusters of similar patients. The machine learning may be unsupervised, with no input as to the type or number of patterns expected. The patterns may be labelled by a system user once they have been produced.

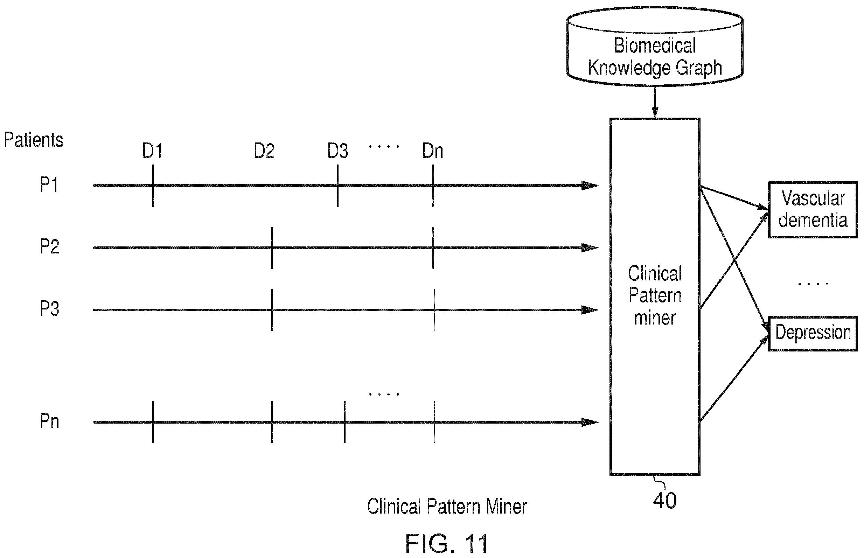

Figure 11 illustrates the inputs and outputs of the clinical pattern miner component. It presents a list of Patients (P1 ... Pn) along with their PCOs (transformed previously from graph representation to timeline representation). Dx represents a particular symptom, diagnosis, treatment, drug, etc. at a particular time. The clinical patient miner identifies (in this case) two group of patients, (1) vascular dementia patients that have vascular dementia as a main diagnosis and (2) depression, patients who have a depression as a main diagnosis. Each of these groups has a subset of the input patients on the left side.

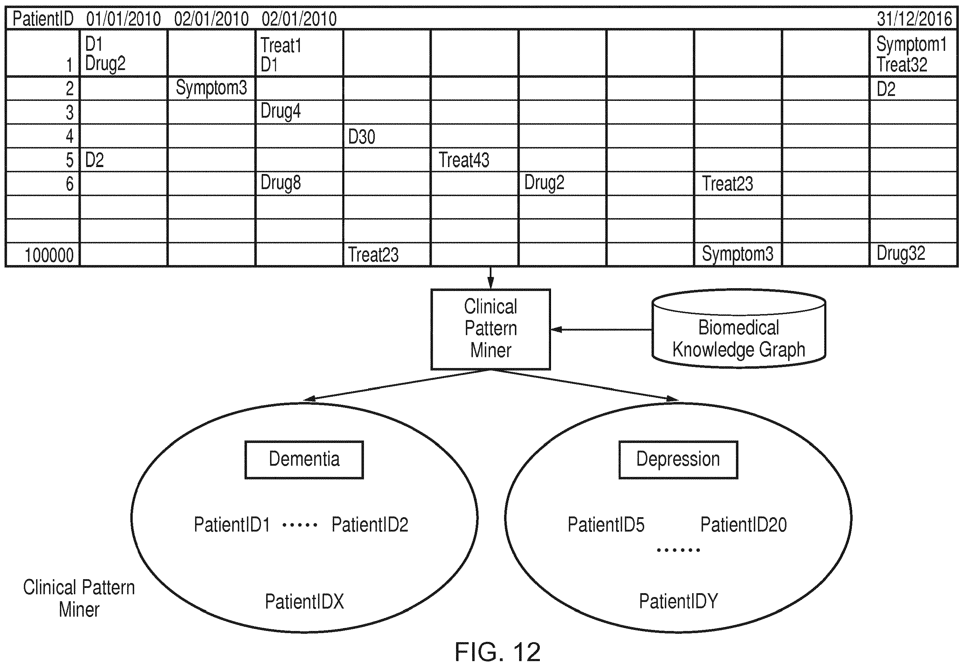

Figure 12 is a more detailed example, illustrating the time representation of PCOs for each patient, with PCOs for patients P1 to P6 and P100 000 shown in detail. The columns indicates the days of the year, of several years.

In this table D stands for diagnosis and Treat for treatment. For example row 1, with Patient ID1 includes a D1 (diagnosis 1), on 01/01/2010 and Drug2 on 01/01/2010.

The pattern miner takes into account

- symptoms
- treatments
- drugs
- diagnoses
- and the frequency of the above

of the patients.

The pattern miner relies on unsupervised learning to create a cluster of patients according to the above information.

As an example the system has produced two clusters/groups of patients as "Dementia" or "Depression". These are the behavioural patterns, in this case clinical patterns, e.g., Dementia and Depression as also shown in Figure 11.

The clinical pattern miner in this example uses input from the biomedical knowledge graph, by taking advantage of the relations among the biomedical entities (symptoms, drugs, treatment, diagnoses, and diseases) on the biomedical knowledge graph. For example, comparing a patient PCO with the biomedical knowledge graph will reveal nodes (entities) that are in both (for instance, the same drug). The data in the PCOs may be supplemented with entities linked to the PCO entities in the biomedical knowledge graph. For example, the nodes neighbouring the same drug node from the biomedical knowledge graph may be added to the PCO if they are not already present. A more sophisticated use may additionally consider a weight of the links and/or entities more than one link away from the entity/node of the PCO.

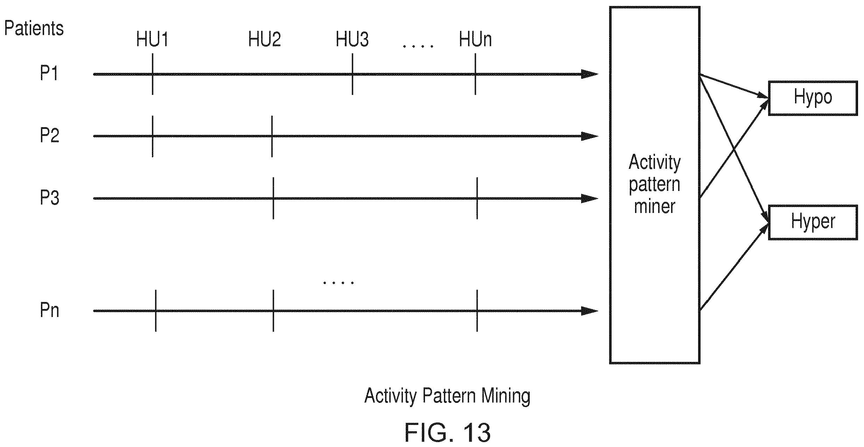

The **Activity Pattern Miner** 320 extracts/identifies patient activity patterns from a set of patients. Basically, this component, by relying on machine learning techniques, extracts and identifies a set of patient activity patterns, i.e., classifies patients based on patient clinical features. Figure 13 illustrates the inputs and outputs of this component. It presents the list of Patients (P1 ... Pn) along with their PAOs (transformed previously from graph representation to timeline representation). Each HUx represents a hospital unit. The activity patient miner identifies (in this case) two group of patients, hypo and hyper groups. Each of these groups has a subset of the input patients on the left side.

For example the component can be extract/identify two groups of patients (1) hypo frequenters, patients who have a low number of hospital visits and (2) hyper frequenters, patients who have a high number of hospital visits. The machine learning may be unsupervised, with no input as to the type or number of patterns expected. The patterns may be labelled by a system user once they have been produced.

It should be noted that the grouped PCO and PAO of a single patient from the set of patients are still linked.

The outcome of this component is a clustered table with the list of patients of a medical institution classified in one or more groups proposed, for the activity and clinical features. Regarding the number of classification labels it is necessary to highlight that there is no prefixed number. In this case there are two (hypo and hyper for activity features) but there could be more depending on the pattern behaviour to be studied, adapting for each case the clinical/activity pattern miner. This outcome is used as pattern to classify the potential behaviour of new patients.

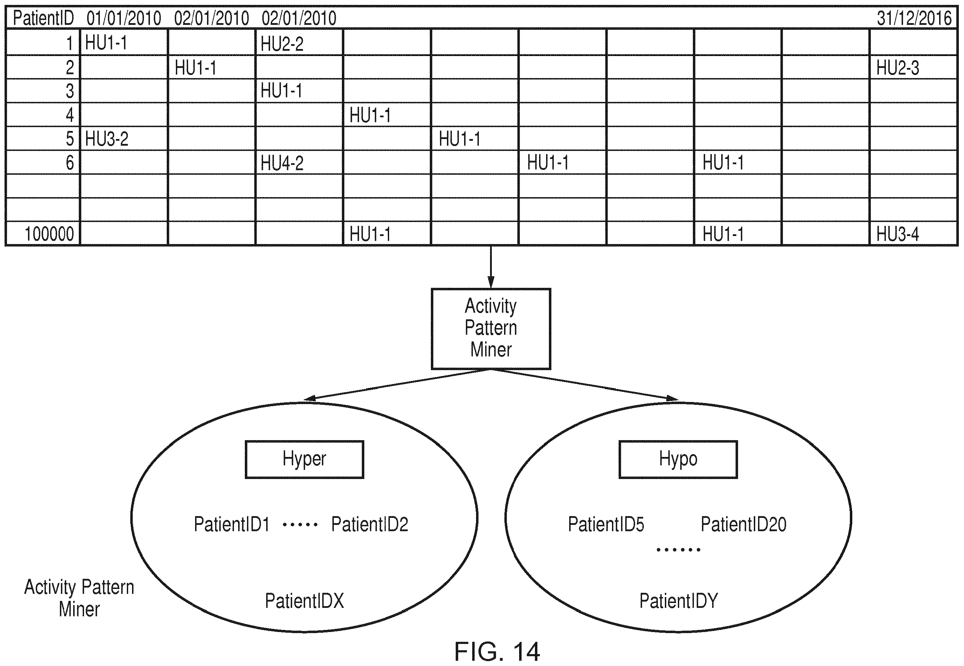

Figure 14 is a more detailed example, illustrating the time representation of PAOs for each patient, with PAOs for patients P1 to P6 and P100 000 shown in detail. The columns indicates the days of the year, of several years.

For example row 1 includes PatientID1 with a hospital visit to the Hospital Unit HU1 one 01/01/2010.

The pattern miner takes into account

- the number of visits

- which hospital units

- and the frequency of the visits

of the patients.

The pattern miner relies on unsupervised learning techniques to create clusters of patients according to the above information. As an example there are two clusters/groups of patients: "Hypo" or "Hyper". These are the behavioural patterns, in this case activity patterns, e.g., hypo and hyper, shown in Figure 13.

The activity pattern miner does not rely on the biomedical knowledge graph

**Patient Behaviour Estimator 50**

Finally, the system can predict/estimate when a patient can be classified in one (or more) identified/extracted behavioural (clinical and activity) patterns, and provide which are the main drivers (underlying nodes) behind those behaviour patterns.

When a new individual patient is to be assessed, the estimator predicts which of the already identified groups (patterns) the new patient fits into, and the estimator also will output what are the features/drivers (which diagnoses, treatments, drugs etc) are the ones that allow the classification of the new patient in the group.

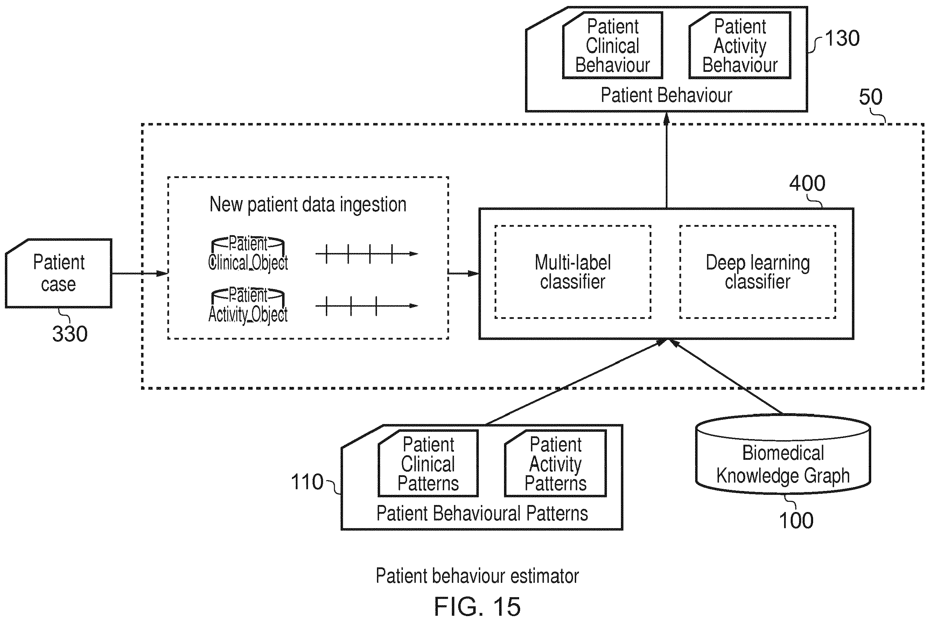

Figure 15 depicts the flow and the main components of this module. The module first retrieves the patient case 330, which includes all the information related to the patient electronic health record, and creates the PCO and PAO along with their temporal representations (for example using the PAO and PCO engines, 200/300 which are not shown). Next, by relying on either multi-label machine learning or deep learning techniques in machine learning component 400, the component classifies the new patient in one of the behavioural (clinical and activity) patterns already identified. The output will be a set of one or more potential patient behaviour groups 130 each associated with a weight, as explained in more detail below.

The machine-learning classifier can be **a random forest classifier.** One classifier may act as both a clinical classifier and an activity classifier, or different classifiers may be used.

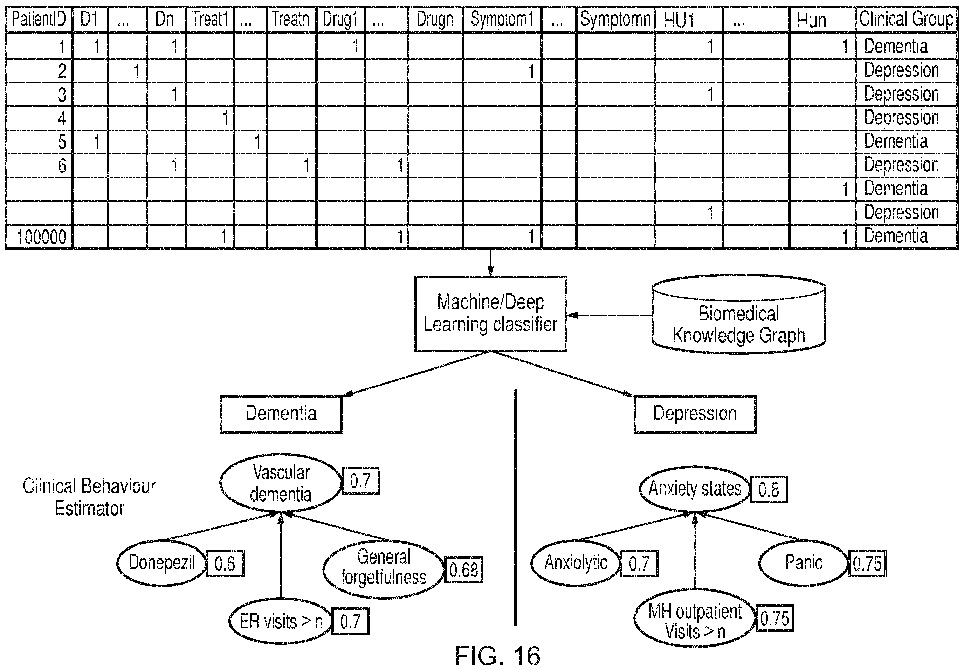

Figure 16 shows an example of a clinical behaviour estimator with patient data and its output, using a classifier. The clinical behaviour estimator takes as input:

- the resultant groups of patients (in this example from the clinical pattern miners)

- a new patient to predict his/her behaviour activity

To this end, a matrix (or other data structure for the PAO and PCO) is used, with the clinical and activity behaviour for each patient. This includes a column for the clinical group (extracted from the clinical pattern miner). The headers of the matrix correspond to the patient IDs and all the treatments, diagnoses, drugs and symptoms, as well as the hospital visits and the clinical group for each patient.

In the example the PatientID 1 has diagnoses D1 and Dn, and Drug1 as well as hospital visit HU1 and he/she was identified as in the "Dementia" group

Next, a machine/deep learning classifier creates a model, in the form of a graph or other relational data structure that assigns weights to biomedical entities for each pattern. As an aside, the same entity can be classified into different patterns so it might have one weight (say 0.3) in one pattern and a different weight (say 0.7) in a second pattern. The model encapsulates which biomedical entities are important for the identified clinical groups. In this example "vascular dementia" has a weight of 0.7 within the dementia group, indicating, for example, the ratio of patients in the population that were classed in this group and had vascular dementia to the total number of patient The model also includes elements of the activity using the same ratio methodology, for example "ER visits greater than n" has a weight of 0.7 within the dementia group.

It is worth mentioning that Machine/Deep learning classifier relies on the biomedical knowledge graph, using the relations among the biomedical entities (symptoms, drugs, treatment, diagnoses, diseases) on the biomedical knowledge graph to supplement the clinical data, as previously explained.

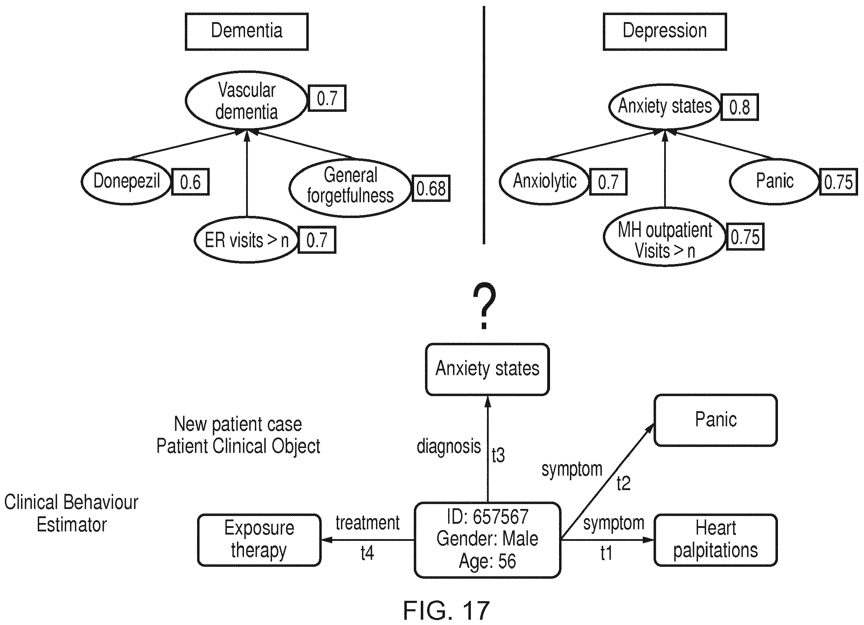

Figure 17 shows the classification of a patient into one of two patient clinical patterns, (i.e., dementia and depression patient features) identified by the clinical pattern miner. Once there are patterns in the model that assigns weights to biomedical entities, the model encapsulates which biomedical entities are important for an identified clinical group and thus a patient with the "vascular dementia" diagnosis has 0.7 probability to be classified as a dementia patient. Equally, a patient with the "panic" symptom has 0.75 probability to be classified as a patient with depression.

The system is able to predict the potential clinical behaviour of the new patient, using comparison of the new patient PCO data with the nodes of the two patterns. The system may also take patient activity data into account.

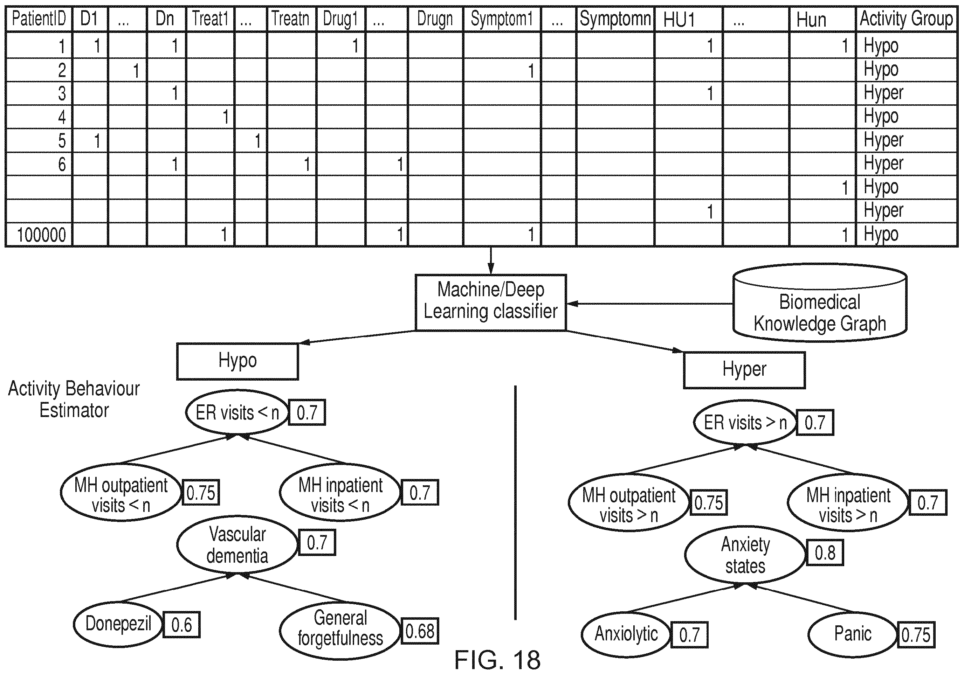

Figure 18 shows an example of an activity behaviour estimator with patient data and its output, using a classifier. The activity behaviour estimator takes as input:

- the resultant groups of patients (in this example from the activity pattern miners)

- a new patient to predict his/her behaviour activity

To this end, a matrix (or other relational data structure) is used with the clinical and activity behaviour for each Patient. This includes a column for the activity group (extracted from the activity pattern miner).

The headers of the matrix corresponds to the patient IDs and all the treatments, diagnoses, drugs and symptoms and activities, and the activity group for each patient. In the example the PatientID 1 has D1, Dn, Drug1 as well as hospital visit HU1, and he/she was identified as Hypo.

Next, a machine/deep learning classifier creates a model, in the form of a graph or other relational data structure, that assigns weights to biomedical entities. The model encapsulates which biomedical entities are important for the identified activity groups, using ratios as previously explained. In this example "vascular dementia" has a weight of 0.7 within the Hypo group. The model also include elements of the activity, for example "less than n" has a weight of 0.7 within the Hypo group.

Again, the machine/deep learning classifier relies on the biomedical knowledge graph, using the relations among the biomedical entities (symptoms, drugs, treatment, diagnoses, diseases) on the biomedical knowledge graph to supplement the data.

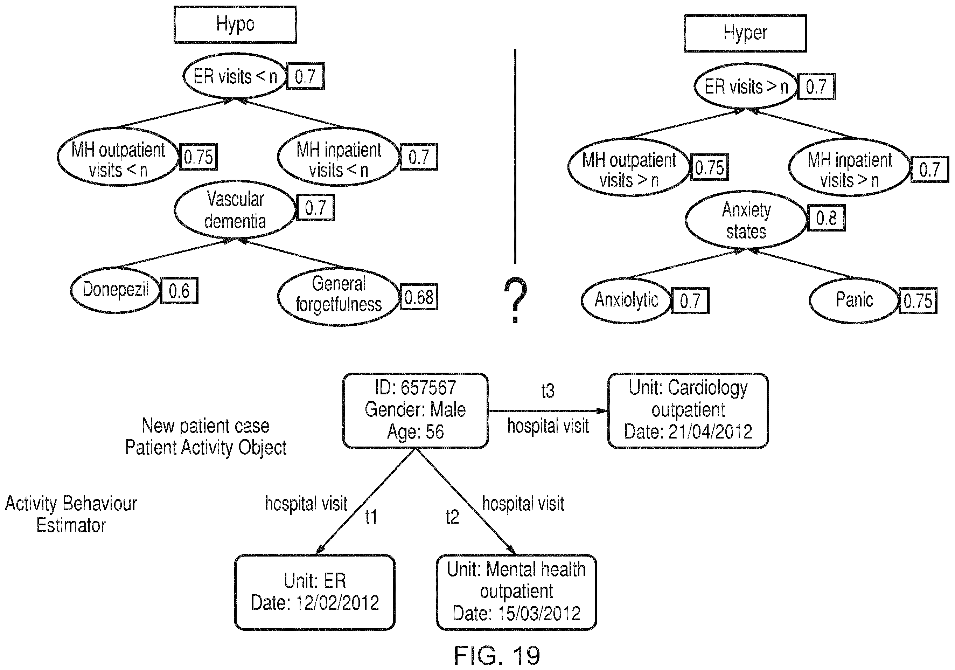

Figure 19 shows the classification of a patient into one of two activity groups/patient activity patterns, i.e., hypo-frequenters and hyper-frequenters. Once there is the model that assigns weights to biomedical entities for each pattern, the model will encapsulate which biomedical entities are important for the identified activity group. In this example "Vascular dementia" has a weight of 0.7 within the Hypo group. Hypo and hyper are simply examples of the patterns/groups identified by the pattern miner, these patterns include the features (drivers) that consists of the nodes along with their probabilities.

The system is able to predict the potential activity behaviour of the new patient, using comparison of the new patient PAO data with the nodes of the two patterns. The system may also take any patient clinical data into account

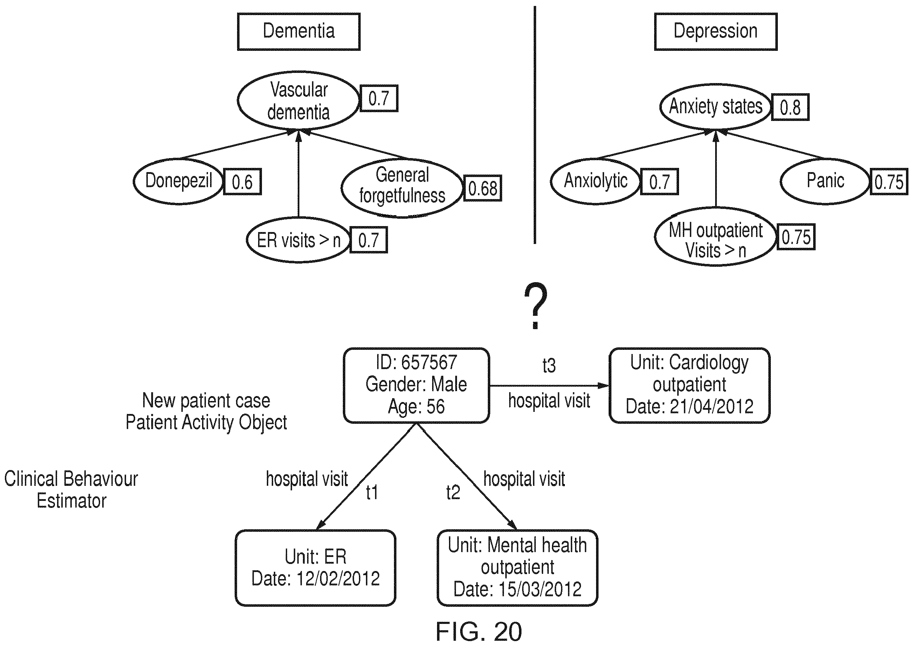

Figure 20 shows the classification of a patient into one of two clinical groups, i.e., dementia and depression, using only patient activity data. This is a concept underlying the invention embodiments. The system is able to predict the clinical behaviour of a new patient using only the activity behaviour of the new patient.

Once the model that assigns weights to biomedical entities has been created, it encapsulates which biomedical entities are important for the identified clinical groups. Within these two groups we can identify health concepts from the biomedical knowledge graph among their relations, and the biomedical knowledge graph has been used to supplement the data. In this example "Vascular dementia" has a weight of 0.7 within the Dementia group. But also there is "ER visits > n" with a weight of 0.7 within the Dementia group. It is thus possible to predict the potential "clinical behaviour" of a new patient, with only her/his activity behaviour. For example purposes, the new patient will be classified into the depression group, because the patient has mental health and ER visits, however, the probability is higher on the mental health visits (0.75). In this case only the activity nodes are available to match the new patient into a pattern.

Of course, these are simplified examples and do not show the full data for the patient population of the full data for the new patient, which is likely to be much more complex. The resultant model from the deep/machine learning classifiers needs to be fed with as much patient data as possible. The greater the number of patients in the population, the better the predictions will be.

The patient behaviour estimator is a meta-predictor, also known as hybrid/combined predictor that makes predictions by organizing and processing the predictions produced by several predictors. The individual predictors take the information for the relevant features from the patterns identified in the model. That is, the new patient is classified into a pattern using its activity nodes that match the activity nodes in that pattern. The matching node(s) in the pattern are used in an individual predictor and the probabilities for all the matching nodes are combined to give an overall probability of classification in the pattern.

Put another way, the probability of the patient being in the pattern (and thus the classification) is calculated by looking at the probability of all the nodes which are both in that pattern and in the patient data. Hence for the example in Figure 20, the only predictor that can work is the one based on hospital visits. For other situations, more predictors will come into play.

The individual predictors available are

• Predictor based on a diagnosis. In this case the prediction is made by checking and reviewing the diagnosis of the patient, and using the probability of any matching diagnosis node in a pattern.

- Predictor based on the drug the patient was taking, and using the probability of any matching drug node in a pattern.
- Predictor based on the symptom of the patient, and using the probability of any matching symptom node in a pattern.
- Predictor based on the treatment the patient is receiving, and using the probability of any matching treatment node in a pattern.
- Predictor based on the hospital visit of the patient, and using the probability of any matching visit node in a pattern. These hospital visits are related to a several hospital over time.

The meta predictor component combines the individual predictors (where available) in order to offer better predicting performance. To this end, the component may adjust the weight given to each one of the predictors. In the following equation

$$Hypo_j = W_d P_d + W_{dr} P_{dr} + W_s P_s + W_t P_t + W_v P_v$$

$Hypo_j$ is the probability for a patient j has the behaviour of hypo-frequenter
$W_d$ is the assigned weight to the predictor based on patient diagnosis
$P_d$ is the prediction based on diagnoses
$W_{dr}$ is the assigned weight to the predictor based on drugs the patient was taking
$P_{dr}$ is the prediction based on drugs the patient was taking
$W_s$ is the assigned weight to the predictor based on symptoms of the patient
$P_s$ is the prediction based on symptoms of the patient.
$W_t$ is the assigned weight to the predictor based on treatments of the patient
$P_t$ is the prediction based on treatments of the patient
$W_t$ is the assigned weight to the predictor based on the number of visits of the patient (there may be different nodes for different types of visits)
$P_t$ is the prediction based on the number of visits of the patient (again, there may be different types of visits)

The assigned weights W are the weights allocated to the nodes
If there is more than one node per category, the node probabilities may be averaged. The weights may be adjusted according to the importance of the different categories, for example to emphasise activity behaviour over clinical behaviour or vice versa.
The overall output of the invention is a decision as to whether a new patient can be classified in one group (from the previously identified groups/patterns from the patient pattern miner) according to his/her clinical behaviour or activity behaviour, with an indication of how closely the new patient PAO/PCO matches with the identified group, based on the weights of the matching nodes

## Benefits

- In a nutshell, estimating the clinical and activity behaviour of a particular patient helps to assign better resources for the treatment of the patient along with the better management of health resources.
- Embodiments of the invention are able to predict the clinical behaviour by only having the activity behaviour.
- Embodiments of the invention may have a high accuracy by use of a theoretical biomedical knowledge graph, and more importantly use of an empirical biomedical knowledge graph based on real patient data.

Some elements of invention embodiments are:

- Biomedical Knowledge Graph, which encodes theoretical and empirical knowledge
- Enhanced Patient Clinical Object, which includes timestamps of the clinical features
- Patient Activity Object, which encodes hospital visits and hospital units over time
- Temporal shaper, which is a component that is able to transform a graph representation into a temporal representation
- Clinical Pattern miner
- Activity Pattern miner
- Patient behaviour estimator, that includes clinical and activity patient behaviour

**Hardware**

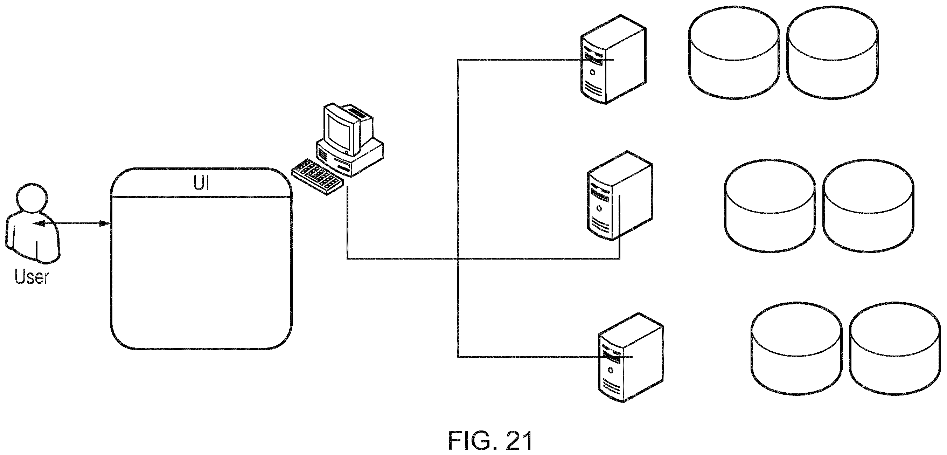

The system for data retrieval as shown in Figure 21 may include a network of computers each responsible for the data processing of a particular type of data (e.g. a computer/server dedicated to the processing of medical literature such as PUBMED4, ATC5, ICD96, ICD107, SNOMED8, etc.). Such a dedicated computer can be physically separate or provided as a virtual server running on shared physical machines. Data may be stored locally on the server/computer and queried by the users who access the system through a user interface UI on a client machine.

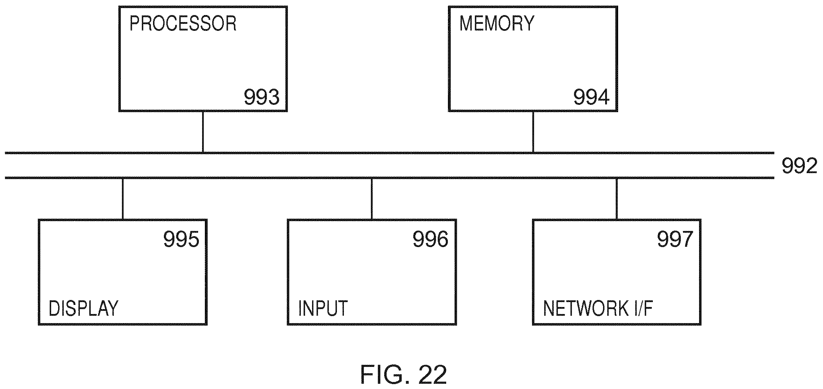

Figure 22 is a block diagram of a computing device, such as a data storage server which embodies the present invention, and which may be used to implement a method of predicting clinical behaviour according to an invention embodiment. The computing device comprises a processor 993, and memory, 994. Optionally, the computing device also includes a network interface 997 for communication with other computing devices, for example with other computing devices of invention embodiments.

The computing device may be one of the servers shown in Figure 21, or may be a separate device. For example, an embodiment may be composed of a network of such computing devices. Optionally, the computing device also includes one or more input mechanisms such as keyboard and mouse 996, and a display unit such as one or more monitors 995. The components are connectable to one another via a bus 992.

The memory 994 stores PCOs and PAOs and may store a completed biomedical knowledge graph 100 shown in figure 3, as well as the empirical and theoretical knowledge graphs used to build the biomedical knowledge graph. It may also store interim results such as the classification of the PCOs/PAOs. The memory may include a computer readable medium, which term may refer to a single medium or multiple media (e.g., a centralized or distributed database and/or associated caches and servers) configured to carry computer-executable instructions or have data structures stored thereon. Computer-executable instructions may include, for example, instructions and data accessible by and causing a general purpose computer, special purpose computer, or special purpose processing device (e.g., one or more processors) to perform one or more functions or operations. Thus, the term "computer-readable storage medium" may also include any medium that is capable of storing, encoding or carrying a set of instructions for execution by the machine and that cause the machine to perform any one or more of the methods of the present disclosure. The term "computer-readable storage medium" may accordingly be taken to include, but not be limited to, solid-state memories, optical media and magnetic media. By way of example, and not limitation, such computer-readable media may include non-transitory computer-readable storage media, including Random Access Memory (RAM), Read-Only Memory (ROM), Electrically Erasable Programmable Read-Only Memory (EEPROM), Compact Disc Read-Only Memory (CD-ROM) or other optical disk storage, magnetic disk storage or other magnetic storage devices, flash memory devices (e.g., solid state memory devices).

The processor 993 is configured to control the computing device and execute processing operations, for example executing code stored in the memory to implement the various different functions of the modules described here and in the claims, including the patient clinical object, PCO, engine, the patient activity object, PAO, engine, the pattern miner(s) and the patient behaviour estimator. The memory 994 stores data (such as PCOs and PAOs, theoretical and empirical medical data and patient data) being read and written by the processor 993. As referred to herein, a processor may include one or more general-purpose processing devices such as a microprocessor, central processing unit, or the like. The processor may include a complex instruction set computing (CISC) microprocessor, reduced instruction set computing (RISC) microprocessor, very long instruction word (VLIW) microprocessor, or a processor implementing other instruction sets or processors implementing a combination of instruction sets. The processor may also include one or more special-purpose processing devices such as an application specific integrated circuit (ASIC), a field programmable gate array (FPGA), a digital signal processor (DSP), network processor, or the like. In one or more embodiments, a processor is configured to execute instructions for performing the operations and steps discussed herein.

The display unit 997 may display a representation of data stored by the computing device and may also display a cursor and dialog boxes and screens enabling interaction between a user and the programs and data stored on the computing device. The input mechanisms 996 may enable a user to input data and instructions to the computing device. For example, the user may input an indication of an individual patient and this may prompt retrieval of the individual's data, and classification of the individual into a clinical or activity pattern, along with generation of the probability associated with such classification.

The network interface (network I/F) 997 may be connected to a network, such as the Internet for retrieval of open data and potentially empirical data and individual patient data and patient population data as shown in Figure 1, and is connectable to other such computing devices via the network. The network I/F 997 may control data input/output from/to other apparatus via the network. Other peripheral devices such as microphone, speakers, printer, power supply unit, fan, case, scanner, trackerball etc may be included in the computing device.

The patient clinical object, PCO, engine 20, may comprise processing instructions stored on a portion of the memory 994, the processor 993 to execute the processing instructions to build the PCO, and a portion of the memory 994 to store input patient population clinical data and resultant PCO data during the execution of the processing instructions. The output of the patient clinical object, PCO, engine, in the form of each finished PCO may be stored on the memory 994 and/or on a connected storage unit.

The patient activity object, PAO, engine 30, may comprise processing instructions stored on a portion of the memory 994, the processor 993 to execute the processing instructions to build the PAO, and a portion of the memory 994 to store input patient population activity data and resultant PAO data during the execution of the processing instructions. The output of the patient clinical object, PAO, engine, in the form of each finished PAO may be stored on the memory 994 and/or on a connected storage unit.

The pattern miner(s) 40, 320 may each comprise processing instructions stored on a portion of the memory 994, the processor 993 to execute the processing instructions, and a portion of the memory 994 to store input data (in the form of PCOs and/or PAOs) and resultant data (grouping the patients into either clinical or activity patterns) during the execution of the processing instructions. The output of the pattern miner(s) and of the full patient behavioural pattern engine 60 (grouping the patients into either clinical or activity patterns) may be stored on the memory 994 and/or on a connected storage unit.

The patient behaviour estimator 50 may comprise processing instructions stored on a portion of the memory 994, the processor 993 to execute the processing instructions, and a portion of the memory 994 to store input data (the patient data and groupings) and resultant data during the execution of the processing instructions. The output of the patient behaviour estimator in the form of classification of an individual/new patient into a group and the associated probability produced by the classifier 400 may be stored on the memory 994 and/or on a connected storage unit.

Methods embodying the present invention may be carried out on a computing device such as that illustrated in Figure 22. Such a computing device need not have every component illustrated in Figure 22, and may be composed of a subset of those components. A method embodying the present invention may be carried out by a single computing device in communication with one or more data storage servers via a network. The computing device may be a data storage itself storing the resultant data of the modules as discussed above.

A method embodying the present invention may be carried out by a plurality of computing devices operating in cooperation with one another. One or more of the plurality of computing devices may be a data storage server storing at least a portion of the resultant data.

### Brief Description of Technical Terms Used

**Patient behaviour/pathway:** The route that a patient follows form the first contact with a hospital through referral to the completion of treatment. The pathway also covers the period from entry into a hospital or a treatment centre until discharge. It is a timeline on which every event relating to treatment can be entered, including consultations, diagnosis, treatment, medication, diet, assessment, teaching and preparing for discharge from hospital.

**Diagnosis:** the process of determining by examination the nature and circumstance of a disease condition from its signs and symptoms.

**Medical treatment:** the management and care of a patient, it includes nursing, psychological intervention and specialist mental health rehabilitation.

**Drugs:** substances that treat or prevent or alleviate the symptoms of a disease.

**Machine Learning:** the subfield of computer science that "gives computers the ability to learn without being explicitly programmed". It explores the study and construction of algorithms that can learn from and make predictions on data.

**Deep Learning:** a branch of machine learning based on a set of algorithms that attempt to model high level abstractions in data.

### Claims

1. A system comprising a processor and memory arranged to predict patient clinical behaviour, the processor being arranged to provide:

    a patient clinical object, PCO, engine to accept historical clinical behaviour of each patient in a population of patients in the form of pieces of clinical information identifying one or more symptoms, treatment, diagnoses and drugs including dates of the symptom, treatment, diagnosis or drug, and to create a PCO stored in the memory for each patient linking a patient identification with a clinical node for each historical piece of clinical information for that patient;
    a patient activity object, PAO, engine to input historical activity behaviour of each patient in the population of patients in the form of pieces of activity information identifying one or more visits to healthcare facilities, including the type of the healthcare facility visited and the date of the visits; and to create a PAO stored in the memory for each patient linking the patient identification with an activity node for each historical piece of activity information for that patient;

a clinical pattern miner, to use machine learning on the patient population PCOs stored in the memory to cluster the patients into two or more clinical patterns according to the PCO of each of the population of patients;

a patient behaviour estimator, to:

input from the memory the PAO and PCO for each of the population of patients clustered within each clinical pattern and to use machine learning to create a model of the clinical patterns, the model including clinical nodes and activity nodes from patients clustered within each clinical pattern; to

input activity behaviour of a new patient in the form of pieces of activity information as to any visits of the new patient to healthcare facilities, including the type of the healthcare facility visited and the date of the visits; to

create a PAO for the new patient linking the new patient identification with an activity node for each piece of activity information for the new patient; to

use machine learning to classify the new patient into one of the clinical patterns by comparing the activity nodes in the new patient PAO with the activity nodes in the pattern; and to

predict new patient clinical behaviour from the clinical nodes of the clinical pattern in which the new patient has been classified.

2.  A system according to claim 1, wherein:

the clinical pattern miner is arranged to:

use a biomedical knowledge graph, the biomedical knowledge graph including symptoms, drugs, treatments, diagnoses and diseases, and links between the symptoms, drugs, treatments, diagnoses, and diseases taken from theoretical and empirical knowledge.

3.  A system according to claim 2, wherein
the clinical pattern miner is arranged to:

supplement nodes on the patient PCOs to provide supplemented PCOs including additional clinical nodes taken from the biomedical knowledge graph, by adding neighbouring nodes from the biomedical knowledge graph to PCO nodes which are already present on both the biomedical knowledge graph and the PCO.

4.  A system according to claim 2 or 3, wherein
the patient behaviour estimator is arranged to input the additional clinical nodes for each of the population of patients clustered within each clinical pattern, wherein each clinical pattern includes the additional clinical nodes from patients clustered within the clinical pattern.

5.  A system according to any of the preceding claims, wherein:

the patient behaviour estimator is arranged to:

label each of the clinical nodes and activity nodes from patients clustered within a clinical pattern with a node weight which is derived from the proportion of patients within the population that include the node and that are clustered within the pattern.

6.  A system according to claim 5, wherein:

the patient behaviour estimator includes a meta predictor arranged to:

use the node weights for nodes of the pattern included within the new patient PAO to derive a combined probability of classification within the pattern.

7.  A system according to claim 6, wherein:

the combined probability is derived by categorising the nodes into diagnoses, drugs, symptoms, treatments and visits and by weighting the contribution of each category with an assigned weight for that category.

8. A system according to any of the preceding claims, further comprising:

an activity pattern miner, to use machine learning on the patient population PAOs to cluster the patients into two or more activity patterns according to the PAO of each of the population of patients; wherein the patient behaviour estimator is additionally arranged to:

use machine learning to create a model of the activity patterns, the model including clinical nodes and activity nodes from patients clustered within each pattern; to
use machine learning to classify the new patient into one of the activity patterns by comparing the activity nodes in the new patient PAO with the activity nodes in the pattern; and to
predict new patient activity behaviour from the activity nodes of the pattern in which the new patient has been classified.

9. A system according to any of the preceding claims, wherein:

the patient behaviour estimator is additionally arranged to:

input clinical behaviour of a new patient in the form of pieces of clinical information identifying one or more symptoms, treatment, diagnoses and drugs including dates of the symptom, treatment, diagnosis or drug; and to create a PCO for the new patient linking the new patient identification with a clinical node for each piece of clinical information for the new patient; and to
additionally use the clinical nodes of the new patient PCO to classify the new patient into a pattern, by additionally comparing the clinical nodes in the new patient PCO with the clinical nodes in the pattern.

10. A system according to claim 9, wherein:

the meta predictor is further arranged to:

additionally use the node weights for nodes of the pattern included within the new patient PCO to derive the combined probability of classification within the pattern.

11. A computer-implemented method of predicting patient clinical behaviour comprising:

inputting historical clinical behaviour of each patient in a population of patients in the form of pieces of clinical information identifying one or more symptoms, treatment, diagnoses and drugs including dates of the symptom, treatment, diagnosis or drug, and creating a PCO for each patient linking a patient identification with a clinical node for each historical piece of clinical information for that patient;
inputting historical activity behaviour of each patient in the population of patients in the form of pieces of activity information identifying one or more visits to healthcare facilities, including the type of the healthcare facility visited and the date of the visits; and creating a PAO for each patient linking the patient identification with an activity node for each historical piece of activity information for that patient;
using machine learning on the patient population PCOs to cluster the patients into two or more clinical patterns according to the PCO of each of the population of patients;
inputting the PAO and PCO for each of the population of patients clustered within each clinical pattern and using machine learning to create a model of each clinical pattern, the model including clinical nodes and activity nodes from patients clustered within the clinical pattern;
inputting activity behaviour of a new patient in the form of pieces of activity information as to any visits of the new patient to healthcare facilities, including the type of the healthcare facility visited and the date of the visits;
creating a PAO for the new patient linking the new patient identification with an activity node for each piece of activity information for the new patient;
using machine learning to classify the new patient into one of the clinical patterns by comparing the activity nodes in the new patient PAO with the activity nodes in the pattern; and
predicting new patient clinical behaviour from the clinical nodes of the clinical pattern in which the new patient has been classified.

12. A computer program which when executed on a computing device carries out the method of claim 11.

13. A non-transitory computer-readable medium storing a computer program according to claim 12.

**Amended claims in accordance with Rule 137(2) EPC.**

1. A system comprising a processor and memory arranged to predict patient clinical behaviour for management of healthcare resources, the processor being arranged to provide:

   a patient health profile engine to accept historical clinical behaviour of each patient in a population of patients in the form of pieces of clinical information identifying one or more symptoms, treatment, diagnoses and drugs including dates of the symptom, treatment, diagnosis or drug, and to create a patient health profile stored in the memory for each patient linking a patient identification with a clinical node for each historical piece of clinical information for that patient;
   a patient activity profile engine to input historical activity behaviour of each patient in the population of patients in the form of pieces of activity information identifying one or more visits to healthcare facilities, including the type of the healthcare facility visited and the date of the visits; and to create a patient activity profile stored in the memory for each patient linking the patient identification with an activity node for each historical piece of activity information for that patient;
   a clinical pattern miner, to use machine learning on the patient population patient health profiles stored in the memory to cluster the patients into two or more clinical patterns according to the patient health profile of each of the population of patients, the clinical patterns defining clusters of similar patients;
   a patient behaviour estimator, to:

   input from the memory the patient activity profile and the patient health profile for each of the population of patients clustered within each clinical pattern and to use machine learning to create a model of the clinical patterns using the input patient activity profiles and patient health profiles, the model including clinical nodes and activity nodes from patients clustered within each clinical pattern; to
   input activity behaviour of a new patient in the form of pieces of activity information as to any visits of the new patient to healthcare facilities, including the type of the healthcare facility visited and the date of the visits; to
   create a patient activity profile for the new patient linking the new patient identification with an activity node for each piece of activity information for the new patient; to
   use machine learning to classify the new patient into one of the clinical patterns by comparing the activity nodes in the patient activity profile of the new patient with the activity nodes in each of the clinical patterns and classifying the new patient into a clinical pattern based on matching activity nodes; and to
   predict new patient clinical behaviour from the clinical nodes of the clinical pattern in which the new patient has been classified to assign resources for the treatment of the new patient.

2. A system according to claim 1, wherein:
   the clinical pattern miner is arranged to:
   use a biomedical knowledge graph, the biomedical knowledge graph including symptoms, drugs, treatments, diagnoses and diseases, and links between the symptoms, drugs, treatments, diagnoses, and diseases taken from theoretical and empirical knowledge.

3. A system according to claim 2, wherein
   the clinical pattern miner is arranged to:
   supplement nodes on the patient health profiles to provide supplemented patient health profiles including additional clinical nodes taken from the biomedical knowledge graph, by adding neighbouring nodes from the biomedical knowledge graph to patient health profile nodes which are already present on both the biomedical knowledge graph and the patient health profile.

4. A system according to claim 2 or 3, wherein
   the patient behaviour estimator is arranged to input the additional clinical nodes for each of the population of patients clustered within each clinical pattern, wherein each clinical pattern includes the additional clinical nodes from patients clustered within the clinical pattern.

5. A system according to any of the preceding claims, wherein:
   the patient behaviour estimator is arranged to:
   label each of the clinical nodes and activity nodes from patients clustered within a clinical pattern with a node weight which is derived from the proportion of patients within the population that include the node and that are clustered within the pattern.

**6.** A system according to claim 5, wherein:
the patient behaviour estimator includes a meta predictor arranged to:
use the node weights for nodes of the pattern included within the patient activity profile of the new patient to derive a combined probability of classification within the pattern.

**7.** A system according to claim 6, wherein:
the combined probability is derived by categorising the nodes into diagnoses, drugs, symptoms, treatments and visits and by weighting the contribution of each category with an assigned weight for that category.

**8.** A system according to any of the preceding claims, further comprising:

an activity pattern miner, to use machine learning on the patient population patient activity profiles to cluster the patients into two or more activity patterns according to the patient activity profile of each of the population of patients; wherein
the patient behaviour estimator is additionally arranged to:

use machine learning to create a model of the activity patterns, the model including clinical nodes and activity nodes from patients clustered within each pattern; to
use machine learning to classify the new patient into one of the activity patterns by comparing the activity nodes in the patient activity profile of the new patient with the activity nodes in the pattern; and to
predict new patient activity behaviour from the activity nodes of the pattern in which the new patient has been classified.

**9.** A system according to any of the preceding claims, wherein:
the patient behaviour estimator is additionally arranged to:

input clinical behaviour of a new patient in the form of pieces of clinical information identifying one or more symptoms, treatment, diagnoses and drugs including dates of the symptom, treatment, diagnosis or drug; and to create a patient health profile for the new patient linking the new patient identification with a clinical node for each piece of clinical information for the new patient; and to
additionally use the clinical nodes of the patient health profile of the new patient to classify the new patient into a pattern, by additionally comparing the clinical nodes in the patient health profile of the new patient with the clinical nodes in the pattern.

**10.** A system according to claim 9, wherein:
the meta predictor is further arranged to:
additionally use the node weights for nodes of the pattern included within the patient health profile of the new patient to derive the combined probability of classification within the pattern.

**11.** A computer-implemented method of predicting patient clinical behaviour for management of healthcare resources, the method comprising:

inputting historical clinical behaviour of each patient in a population of patients in the form of pieces of clinical information identifying one or more symptoms, treatment, diagnoses and drugs including dates of the symptom, treatment, diagnosis or drug, and creating a patient health profile for each patient linking a patient identification with a clinical node for each historical piece of clinical information for that patient;
inputting historical activity behaviour of each patient in the population of patients in the form of pieces of activity information identifying one or more visits to healthcare facilities, including the type of the healthcare facility visited and the date of the visits; and creating a patient activity profile for each patient linking the patient identification with an activity node for each historical piece of activity information for that patient;
using machine learning on the patient population patient health profiles to cluster the patients into two or more clinical patterns according to the patient health profile of each of the population of patients, the clinical patterns defining clusters of similar patients;
inputting the patient activity profile and the patient health profile for each of the population of patients clustered within each clinical pattern and using machine learning to create a model of each clinical pattern using the input patient activity profiles and patient health profiles, the model including clinical nodes and activity nodes from patients clustered within the clinical pattern;
inputting activity behaviour of a new patient in the form of pieces of activity information as to any visits of the new patient to healthcare facilities, including the type of the healthcare facility visited and the date of the visits;
creating a patient activity profile for the new patient linking the new patient identification with an activity node for each piece of activity information for the new patient;
using machine learning to classify the new patient into one of the clinical patterns by comparing the activity nodes in the patient activity profile of the new patient with the activity nodes in each of the clinical patterns and classifying the new patient into a clinical pattern based on matching activity nodes; and
predicting new patient clinical behaviour from the clinical nodes of the clinical pattern in which the new patient has been classified to assign resources for the treatment of the new patient.

**12.** A computer program which when executed on a computing device carries out the method of claim 11.

**13.** A non-transitory computer-readable medium storing a computer program according to claim 12.

FIG. 1

Create PCO $\quad$ S10

Create PAO $\quad$ S20

Use machine learning on PCOs to cluster into clinical patterns $\quad$ S30

Create a model of each clinical patterns using activity and clinical nodes from patients clustered within clinical patterns $\quad$ S40

Input activity behaviour of individual patient $\quad$ S50

Use machine learning to predict individual patient clinical behaviour by comparing the individual's activity behaviour with the patterns $\quad$ S60

FIG. 2

FIG. 3

Main system components

- Patient case — 120
- Patient Behaviour Estimator — 50
- Patient behaviour — 130
- Biomedical Knowledge Graph — 100
- Biomedical Knowledge Graph Engine — 70
- Patient Behavioural Patterns — 110
- Patient Behaviour Pattern Engine — 60
- Clinicians knowledge — 90
- Open Data: ICD9/10, CHEBI /ATC, PUB-MED, SNOMED /MESH, HDO
- Patient Data — 80: Historical Clinical Data, Clinical Guidelines & protocols

FIG. 4

Biomedical Theoretical Knowledge graph

FIG. 5

Biomedical Empirical Knowledge Graph

FIG. 6

Biomedical Knowledge Graph

FIG. 7

FIG. 8

Patient

P1

Heart palpitations    Panic    Anxiety states    Exposure therapy

t1    t2    t3    t4

Panic

Heart palpitations

symptom t2

symptom t1

Anxiety states

diagnosis t3

ID: 657567
Gender: Male
Age: 56

treatment t4

Exposure therapy

FIG. 9

28

EP 3 382 584 A1

FIG. 10

FIG. 11

EP 3 382 584 A1

| PatientID | 01/01/2010 | 02/01/2010 | 02/01/2010 | | | | | | 31/12/2016 |
|---|---|---|---|---|---|---|---|---|---|
| 1 | D1 Drug2 | | Treat1 D1 | | | | | | Symptom1 Treat32 |
| 2 | | Symptom3 | | | | | | | D2 |
| 3 | | | Drug4 | | | | | | |
| 4 | | | | D30 | | | | | |
| 5 | D2 | | | | Treat43 | | | | |
| 6 | | | Drug8 | | | Drug2 | | Treat23 | |
| | | | | | | | | | |
| | | | | | | | | | |
| 100000 | | | | Treat23 | | | | Symptom3 | Drug32 |

Clinical Pattern Miner

Biomedical Knowledge Graph

Dementia

PatientID1 ····· PatientID2

PatientIDX

Depression

PatientID5    PatientID20

······

PatientIDY

Clinical Pattern Miner

FIG. 12

Activity Pattern Mining

FIG. 13

EP 3 382 584 A1

| PatientID | 01/01/2010 | 02/01/2010 | 02/01/2010 | | | | | | 31/12/2016 |
|---|---|---|---|---|---|---|---|---|---|
| 1 | HU1-1 | | HU2-2 | | | | | | |
| 2 | | HU1-1 | | | | | | | HU2-3 |
| 3 | | | HU1-1 | | | | | | |
| 4 | | | | HU1-1 | | | | | |
| 5 | HU3-2 | | | | HU1-1 | | | | |
| 6 | | | HU4-2 | | | HU1-1 | | HU1-1 | |
| | | | | | | | | | |
| | | | | | | | | | |
| 100000 | | | HU1-1 | | | | | HU1-1 | HU3-4 |

Activity Pattern Miner

Hyper

PatientID1 ····· PatientID2

PatientIDX

Hypo

PatientID5      PatientID20

······

PatientIDY

Activity Pattern Miner

FIG. 14

EP 3 382 584 A1

FIG. 15

Patient behaviour estimator

| PatientID | D1 | ... | Dn | Treat1 | ... | Treatn | Drug1 | ... | Drugn | Symptom1 | ... | Symptomn | HU1 | ... | Hun | Clinical Group |
|---|---|---|---|---|---|---|---|---|---|---|---|---|---|---|---|---|
| 1 | 1 | | 1 | | | | 1 | | | | | | 1 | | 1 | Dementia |
| 2 | | 1 | | | | | | | | 1 | | | | | | Depression |
| 3 | | | 1 | | | | | | | | | | 1 | | | Depression |
| 4 | | | | 1 | | | | | | | | | | | | Depression |
| 5 | 1 | | | | 1 | | | | | | | | | | | Dementia |
| 6 | | | 1 | | | | 1 | 1 | | | | | | | | Depression |
| | | | | | | | | | | | | | | | 1 | Dementia |
| | | | | | | | | | | | | | | 1 | | Depression |
| 100000 | | | | 1 | | | | | 1 | 1 | | | | | 1 | Dementia |

FIG. 16

FIG. 17

EP 3 382 584 A1

| PatientID | D1 | ... | Dn | Treat1 | ... | Treatn | Drug1 | ... | Drugn | Symptom1 | ... | Symptomn | HU1 | ... | Hun | Activity Group |
|---|---|---|---|---|---|---|---|---|---|---|---|---|---|---|---|---|
| 1 | 1 | | 1 | | | 1 | | | | | | | 1 | | 1 | Hypo |
| 2 | | 1 | | | | | | | 1 | | | | | | | Hypo |
| 3 | | | 1 | | | | | | | | | | 1 | | | Hyper |
| 4 | | | | 1 | | | | | | | | | | | | Hypo |
| 5 | 1 | | | | 1 | | | | | | | | | | | Hyper |
| 6 | | | 1 | | | | 1 | | 1 | | | | | | | Hyper |
| | | | | | | | | | | | | | | | 1 | Hypo |
| | | | | | | | | | | | | | 1 | | | Hyper |
| 100000 | | | | 1 | | | | | 1 | | 1 | | | | 1 | Hypo |

Machine/Deep Learning classifier

Biomedical Knowledge Graph

Hypo

Hyper

Activity Behaviour Estimator

ER visits < n  0.7

MH outpatient visits < n  0.75

MH inpatient visits < n  0.7

Vascular dementia  0.7

Donepezil  0.6

General forgetfulness  0.68

ER visits > n  0.7

MH outpatient visits > n  0.75

MH inpatient visits > n  0.7

Anxiety states  0.8

Anxiolytic  0.7

Panic  0.75

FIG. 18

FIG. 19

Dementia

Vascular dementia [0.7]

Donepezil [0.6]

ER visits > n [0.7]

General forgetfulness [0.68]

Depression

Anxiety states [0.8]

Anxiolytic [0.7]

MH outpatient Visits > n [0.75]

Panic [0.75]

?

New patient case
Patient Activity Object

Clinical Behaviour
Estimator

ID: 657567
Gender: Male
Age: 56

t3
hospital visit

Unit: Cardiology
outpatient
Date: 21/04/2012

hospital visit
t1

t2
hospital visit

Unit: ER
Date: 12/02/2012

Unit: Mental health
outpatient
Date: 15/03/2012

FIG. 20

FIG. 21

User

UI

FIG. 22

**Europäisches Patentamt**

**European Patent Office**

**Office européen des brevets**

# EUROPEAN SEARCH REPORT

Application Number

EP 17 16 4007

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2016/125159 A1 (LING BRUCE X [US] ET AL) 5 May 2016 (2016-05-05)<br>* abstract; figures 1-11 *<br>* paragraphs [0017] - [0018] *<br>* paragraphs [0036] - [0038] *<br>* paragraphs [0043] - [0045] *<br>* paragraphs [0062] - [0064] *<br>* paragraphs [0070] - [0076] *<br>* paragraphs [0080] - [0081] *<br>----- | 1-13 | INV.<br>G06F19/00 |
| X | US 2011/295621 A1 (FAROOQ FAISAL [US] ET AL) 1 December 2011 (2011-12-01)<br>* abstract; claims 1,2,8; figures 1-4 *<br>* paragraphs [0018] - [0020] *<br>* paragraphs [0025] - [0026] *<br>* paragraphs [0034] - [0037] *<br>* paragraph [0045] *<br>* paragraphs [0050] - [0051] *<br>* paragraph [0079] *<br>* paragraphs [0086] - [0087] *<br>* paragraphs [0092] - [0095] *<br>----- | 1-13 | |
| X | US 2015/161346 A1 (HU JIANYING [US] ET AL) 11 June 2015 (2015-06-11)<br>* abstract; figures 1-7 *<br>* paragraphs [0018] - [0019] *<br>* paragraphs [0030] - [0031] *<br>* paragraphs [0035] - [0036] *<br>* paragraphs [0039] - [0042] *<br>* paragraphs [0072] - [0076] *<br>----- | 1-13 | TECHNICAL FIELDS SEARCHED (IPC)<br>G06F |

The present search report has been drawn up for all claims

| Place of search | Date of completion of the search | Examiner |
|---|---|---|
| Munich | 7 September 2017 | Filloy García, E |

EPO FORM 1503 03.82 (P04C01)

# EP 3 382 584 A1

## ANNEX TO THE EUROPEAN SEARCH REPORT
## ON EUROPEAN PATENT APPLICATION NO.

EP 17 16 4007

This annex lists the patent family members relating to the patent documents cited in the above-mentioned European search report.
The members are as contained in the European Patent Office EDP file on
The European Patent Office is in no way liable for these particulars which are merely given for the purpose of information.

07-09-2017

| Patent document cited in search report | | Publication date | Patent family member(s) | | Publication date |
|---|---|---|---|---|---|
| US 2016125159 | A1 | 05-05-2016 | US 2016125159 A1<br>WO 2016073776 A1 | | 05-05-2016<br>12-05-2016 |
| US 2011295621 | A1 | 01-12-2011 | NONE | | |
| US 2015161346 | A1 | 11-06-2015 | CN 104699939 A<br>DE 102014116177 A1<br>US 2015161346 A1 | | 10-06-2015<br>11-06-2015<br>11-06-2015 |

EPO FORM P0459

For more details about this annex : see Official Journal of the European Patent Office, No. 12/82